# EUROPEAN PATENT SPECIFICATION

(11) **EP 2 035 451 B1**
(45) Date of publication and mention of the grant of the patent: **05.05.2010**
(21) Application number: 07765481.2
(22) Date of filing: 19.06.2007
(51) Int. Cl.: C07K 14/605, A61K 38/26

(54) **INSULINOTROPIC PEPTIDE SYNTHESIS**
SYNTHESE EINES INSULINOTROPEN PEPTIDS
SYNTHÈSE DE PEPTIDES INSULINOTROPES

(30) Priority: 23.06.2006 US 815919 P
(43) Date of publication of application: 18.03.2009
(73) Proprietor: F.HOFFMANN-LA ROCHE AG, 4070 Basel (CH)
(72) Inventor: CHEN, Lin, Superior, Colorado 80027 (US); HAN, Yeun-Kwei, Louisville, Colorado 80027 (US); ROBERTS, Christopher R., Berthoud, Colorado 80513 (US)
(74) Representative: Klostermeyer-Rauber, Dörte
(86) International application number: PCT/EP2007/056052
(87) International publication number: WO 2007/147816

(56) References cited:
- WO-A-00/34331
- WO-A-2004/094461
- US-B1- 6 514 500
- ANDERSSON L ET AL: "Large-scale synthesis of peptides." BIOPOLYMERS 2000, vol. 55, no. 3, 2000, pages 227-250, XP002457690 ISSN: 0006-3525 cited in the application
- SAMPSON W R ET AL: "The synthesis of 'difficult' peptides using 2-hydroxy-4-methoxybenzyl or pseudoproline amino acid building blocks: A comparative study" JOURNAL OF PEPTIDE SCIENCE, JOHN WILEY AND SONS LTD, GB, vol. 5, no. 9, 1999, pages 403-409, XP002419351 ISSN: 1075-2617
- GARCÍA-MARTÍN FAYNA ET AL: "The synergy of ChemMatrix resin and pseudoproline building blocks renders RANTES, a complex aggregated chemokine." BIOPOLYMERS 2006, vol. 84, no. 6, 2006, pages 566-575, XP002457691 ISSN: 0006-3525
- CREMER GAËLLE-ANNE ET AL: "Combining a polar resin and a pseudo-proline to optimize the solid-phase synthesis of a 'difficult sequence'." JOURNAL OF PEPTIDE SCIENCE : AN OFFICIAL PUBLICATION OF THE EUROPEAN PEPTIDE SOCIETY JUN 2006, vol. 12, no. 6, June 2006 (2006-06), pages 437-442, XP002457692 ISSN: 1075-2617

## Description

The invention relates to methods for preparing insulinotropic peptides, particularly glucagon-like peptide-1 (GLP-1) and counterparts thereof, using solid- and solution-phase processes. The present invention further relates to intermediate peptide fragments that can be used in these methods.

Many methods for peptide synthesis are described in the literature (for example, *see* U.S. Patent No. 6,015,881; Mergler et al. (1988) Tetrahedron Letters 29:4005-4008; Mergler et al. (1988) Tetrahedron Letters 29:4009-4012; Kamber et al. (eds), Peptides, Chemistry and Biology, ESCOM, Leiden (1992) 525-526; Riniker et al. (1993) Tetrahedron Letters 49:9307-9320; Lloyd-Williams et al. (1993) Tetrahedron Letters 49:11065-11133; and Andersson et al. (2000) Biopolymers 55:227-250. The various methods of synthesis are distinguished by the physical state of the phase in which the synthesis takes place, namely liquid phase or solid phase.

In solid phase peptide synthesis (SPPS), an amino acid or peptide group is bound to a solid support resin. Then, successive amino acids or peptide groups are attached to the support-bound peptide until the peptide material of interest is formed. The support-bound peptide is then typically cleaved from the support and subject to further processing and/or purification. In some cases, solid phase synthesis yields a mature peptide product; in other cases the peptide cleaved from the support (i.e., a "peptide intermediate fragment") is used in the preparation of a larger, mature peptide product.

Peptide intermediate fragments generated from solid phase processes can be coupled together in the solid phase or in a liquid phase synthetic process (herein referred to as "solution phase synthesis"). Solution phase synthesis can be particularly useful in cases where the synthesis of a useful mature peptide by solid phase is either impossible or not practical. For example, in solid phase synthesis, longer peptides eventually may adopt an irregular conformation while still attached to the solid support, making it difficult to add additional amino acids or peptide material to the growing chain. As the peptide chain becomes longer on the support resin, the efficiency of process steps such as coupling and deprotection may be compromised. This, in turn, can result in longer processing times to compensate for these problems, in addition to incremental losses in starting materials, such as activatable amino acids, co-reagents, and solvents. These problems can increase as the length of the peptide increases.

Therefore, it is relatively uncommon to find mature peptides of greater than 30 amino acids in length synthesized in a single fragment using only a solid phase procedure. Instead, individual fragments may be separately synthesized on the solid phase, and then coupled in the solid and/or solution phase to build the desired peptide product. This approach requires careful selection of fragment candidates. While some general principles can guide fragment selection, quite often empirical testing of fragment candidates is required. Fragment strategies that work in one context may not work in others. Even when reasonable fragment candidates are uncovered, process innovations may still be needed for a synthesis strategy to work under commercially reasonable conditions. Therefore, peptide synthesis using hybrid schemes are often challenging, and in many cases it is difficult to predict what problems are inherent in a synthesis scheme until the actual synthesis is performed.

In solution phase coupling, two peptide intermediate fragments, or a peptide intermediate fragment and a reactive amino acid, are coupled in an appropriate solvent, usually in the presence of additional reagents that promote the efficiency and quality of the coupling reaction. The peptide intermediate fragments are reactively arranged so the N-terminal of one fragment becomes coupled to the C-terminal of the other fragment, or *vice versa*. In addition, side chain protecting groups, which are present during solid phase synthesis, are commonly retained on the fragments during solution phase coupling to ensure the specific reactivity of the terminal ends of the fragments. These side chain protecting groups are typically not removed until a mature peptide has been formed.

Modest improvements in one or more steps in the overall synthetic scheme can amount to significant improvements in the preparation of the mature peptide. Such improvements can lead to a large overall saving in time and reagents, and can also significantly improve the purity and yield of the final product.

While the discussion of the importance of improvements in hybrid synthesis is applicable to any sort of peptide produced using these procedures, it is of particular import in the context of peptides that are therapeutically useful and that are manufactured on a scale for commercial medical use. Synthesis of larger biomolecular pharmaceuticals, such as therapeutic peptides, can be very expensive. Because of the cost of reagents, synthesis time, many synthesis steps, in addition to other factors, very small improvements in the synthetic process of these larger biomolecular pharmaceuticals can have a significant impact on whether it is even economically feasible to produce such a pharmaceutical. Such improvements are necessary due to these high production costs for larger biomolecular pharmaceuticals as supported by the fact that, in many cases, there are few, if any, suitable therapeutic alternatives for these types of larger biomolecular pharmaceuticals.

This is clearly seen in the case of the glucagon-like peptide-1 (GLP-1) and its counterparts. These peptides have been implicated as possible therapeutic agents for the treatment of type 2 non-insulin-dependent diabetes mellitus as well as related metabolic disorders, such as obesity. Gutniak, M.K., et al., Diabetes Care 1994:17:1039-44.

Lopez et al. determined that native GLP-1 was 37 amino acid residues long. Lopez, L. C., et al., Proc. Natl. Acad. Sci. USA., 80:5485-5489 (1983). This determination was confirmed by the work of Uttenthal, L. O., et al., J. Clin. Endocrinal. Metabol., 61:472-479 (1985). Native GLP-1 may be represented by the notation GLP-1 (1-37). This notation indicates that the peptide has all amino acids from 1 (N-terminus) through 37 (C-terminus). Native GLP-1 has the amino acid sequence according to SEQ ID NO. 1:
HDEFERHAEGTFTSDVSSYLEGQAAKEFIAWLVKGRG

It has been reported that native GLP-1(1-37) is generally unable to mediate insulin biosynthesis, but biologically important fragments of this peptide do have insulinotropic properties. For example, the native 31-amino acid long peptide GLP-1 (7-37) according to SEQ ID NO. 2:
HAEGTFTSDVSSYLEGQAAKEFIAWLVKGRG
   is insulinotropic and has the amino acids from the 7 (N terminus) to the 37 (C terminus) position of native GLP-1. GLP-1 (7-37) has a terminal glycine. When this glycine is absent, the resultant peptide is still insulinotropically active and is referred to as GLP-1 (7-36) according to SEQ ID NO. 3:
HAEGTFTSDVSSYLEGQAAKEFIAWLVKGR
   GLP-1 (7-36) often exists with the C-terminal arginine in amidated form, and this form may be represented by the notation GLP-1 (7-36)-NH2.
   GLP-1(1-37) generally is converted into an insulinotropically active counterpart thereof in vivo. For instance, GLP-1 (1-37) is naturally converted to GLP-1 (7-37) in vivo. This peptide, in turn, can also undergo additional processing by proteolytic removal of the C-terminal glycine to produce GLP-1 (7-36), which often exists in the amidated form GLP-1(7-36)-NH₂. Accordingly, therapeutic treatments may involve administration of GLP-1 (1-37) or a counterpart thereof, with the expectation that an insulinotropically active derivative thereof forms in vivo. More commonly, however, therapeutic treatments under investigation involve administration of the insulinotropically active GLP-1 fragments themselves.

According to US 6,887,849, the insulinotropic activity of GLP-1(7-37), GLP-1(7-36) and GLP-1(7-36)-NH₂ appears to be specific for the pancreatic beta cells, where these peptides appear to induce biosynthesis of insulin. This makes these peptides and pharmaceutically acceptable counterparts thereof useful in the study of the pathogenesis of adult onset diabetes mellitus, a condition characterize by hyperglycemia in which the dynamics of insulin secretion are abnormal. Moreover, these glucagon-like peptides would be useful in the therapy and treatment of this disease, and in the therapy and treatment of hyperglycemia. According to EP 1137667B1, these peptides or pharmaceutically acceptable counterparts thereof may also be useful for treating other types of diabetes, obesity, glucagonomas, secretory disorders of the airway, metabolic disorder, arthritis, osteoporosis, central nervous system disease, restenosis, neurodegenerative disease, renal failure, congestive heart failure, neophrotic syndrome, cirrhosis, pulmonary edema, hypertension, and/or disorders where a reduction in food intake is desired.

Native GLP-1(1-37) and the native, insulinotropically active counterparts thereof according to SEQ ID NO. 1 through 3 are metabolically unstable, having a plasma half-life of only 1 to 2 minutes in vivo. Exogenously administered GLP-1 also is rapidly degraded. This metabolic instability has limited the therapeutic potential of native GLP-1 and native fragments thereof.

Synthetic counterparts of the GLP-1 peptides with improved stability have been developed. For instance, the peptide according to SEQ ID NO. 4 is described in EP 1137667 B1:
HAibEGTFTSDVSSYLEGQAAKEFIAWLVKAibR

This peptide is similar to the native GLP-1(7-36), except that the achiral residue of alpha-aminoisobutyric acid (shown schematically by the abbreviation Aib) appears at the 8 and 35 positions in place of the corresponding native amino acids at these positions. The achiral alpha-aminoisobutric acid also is known as methylalanine. This peptide may be designated by the formula (Aib ^{8,35})GLP-1(7-36)-NH₂.

EP 1137667 states that the peptide according to SEQ ID NO: 4 and its counterparts can be built as a single fragment using solid phase techniques. The single fragment synthesis approach suggested by EP 1137667 is problematic. As one issue, this approach may lead to high levels of epimerization in the final amino acid coupling, e.g., histidine in the case of (Aib ^{8,35}) GLP-1 (7-36) for instance. Additionally, impurities may be hard to remove during chromatographic purification, and the yield may tend to be too low. Consequently, improved strategies for synthesizing peptides according to SEQ ID NO. 4 are needed in order to be able to manufacture this peptide and counterparts thereof in commercially acceptable yields, purities, and quantities.

In addition to these concerns, issues relating to product recovery and product purity for the large-scale production of peptides, as well as reagent handling, storage and disposal, can greatly impact the feasibility of the peptide synthesis scheme. Thus, there is a continuing need for peptide synthesis processes capable of efficiently producing peptide materials of commercial interest in large batch quantities with improved yields.

The present invention relates to the preparation of insulinotropic peptides, that are synthesized using a solid and solution phase ("hybrid") approach. Generally, the approach includes synthesizing three different peptide intermediate fragments using solid phase chemistry. Solution phase chemistry is then used to add additional amino acid material to one of the fragments. The fragments are then coupled together in the solid and solution phases. The use of a pseudoproline in one of the fragments eases the solid phase synthesis of that fragment and also eases the subsequent solution phase coupling of this fragment to other fragments. The present invention is very useful for forming insulinotropic peptides such as GLP-1, GLP-1(7-36) and natural and non-natural counterparts of these, particularly GLP-1 (7-36) and its natural and non-natural counterparts.

In one aspect, the present invention relates to a method of making an insulinotropic peptide, comprising the steps of:
a) preparing a peptide fragment including the amino acid sequence HX⁸EX¹⁰ (SEQ ID NO. 6) wherein X⁸ and X¹⁰ are each residues of an achiral amino acid, or said fragment is a counterpart thereof including the X⁸ and X¹⁰ residues, each of H, E, X⁸ and X¹⁰ optionally including side chain protection; and
b) incorporating the peptide fragment into an insulinotropic peptide.

Preferably, X⁸ is an amino acid residue corresponding to methyl alanine (Aib). X¹⁰ is preferably an amino acid residue corresponding to glycine.

In a further aspect, the invention relates to a peptide fragment having the amino acid sequence HX⁸EX¹⁰ (SEQ ID NO. 6), wherein X⁸ and X¹⁰ are each residues of an achiral amino acid, each of H, E, X⁸ and X¹⁰ optionally including side chain protection. Preferably, X⁸ is an amino acid residue corresponding to Aib and X¹⁰ is an amino acid residue corresponding to glycine.

In another aspect, the present invention relates to a method of making an insulinotropic peptide, comprising the steps of:
a) preparing a peptide fragment or a counterpart thereof including the amino acid sequence TFTSDVX¹⁷⁻¹⁸YLEG (SEQ. ID No. 8) wherein the residue denoted by the symbol X¹⁷⁻¹⁸ is a dipeptide residue of a pseudoproline; and
b) incorporating the peptide fragment into an insulinotropic peptide.

In another aspect, the present invention relates to a peptide or a counterpart thereof including the amino acid sequence TFTSDVX¹⁷⁻¹⁸YLEG (SEQ. ID No. 8) wherein the residue denoted by the symbol X¹⁷⁻¹⁸ is a dipeptide residue of a pseudoproline; said amino acid residues optionally including side chain protection.

In a further aspect, the present invention relates to method of making an insulinotropic peptide according to claim 1, comprising the step of:
a) coupling the first peptide fragment including the amino acid sequence HX⁸EX¹⁰ (SEQ ID NO. 6), wherein X⁸ and X¹⁰ are each residues of an achiral amino acid, each of H and E optionally including side chain protection, to the second peptide fragment including the amino acid sequence TFTSDVX¹⁷⁻¹⁸YLEG (SEQ ID NO. 8),
   wherein the residue denoted by the symbol X¹⁷⁻¹⁸ is a dipeptide residue of a pseudoproline, said amino acid residues of the sequence optionally including side chain protection, to provide a third peptide fragment including the amino acid sequence HX⁸EX¹⁰ TFTSDVX¹⁷⁻¹⁸YLEG (SEQ ID NO. 11), said amino acid residues of the sequence optionally including side chain protection; and
b) incorporating the peptide fragment into an insulinotropic peptide.

In another aspect, the present invention relates to a method of making an insulinotropic peptide, comprising the steps of:
a) preparing a peptide fragment or counterpart thereof including the amino acid sequence QAAKEFIAWLVKX³⁵ (SEQ ID NO. 9), wherein X³⁵ is a residue of an achiral amino acid, said residues of the sequence optionally including side chain protection; and
b) incorporating the peptide fragment into an insulinotropic peptide.

In another aspect, the present invention relates to a method of making an insulinotropic peptide, comprising one or more of the steps of:
a) providing a first peptide fragment including the amino acid sequence HX⁸EX¹⁰ (SEQ ID NO. 6), wherein X⁸ and X¹⁰ are each residues of an achiral amino acid, each of H and E optionally including side chain protection;
b) providing a second peptide fragment including the amino acid sequence TFTSDVX¹⁷⁻¹⁸YLEG (SEQ ID NO. 8) wherein the residue denoted by the symbol X¹⁷⁻¹⁸ is a dipeptide residue of a pseudoproline, said amino acid residues of the sequence optionally including side chain protection;
c) coupling the first fragment to the second fragment to provide a third peptide fragment including the amino acid sequence HX⁸EX¹⁰TFTSDVX¹⁷⁻¹⁸YLEG (SEQ ID NO. 11), said amino acid residues of the sequence optionally including side chain protection;
d) providing a fourth peptide fragment including the amino acid sequence QAAKEFIAWLVKX³⁵ (SEQ ID NO. 9), wherein X³⁵ is a residue of an achiral amino acid, said amino acid residues of the sequence optionally including side chain protection;
e) coupling the fourth peptide fragment to arginine in order to provide a fifth peptide fragment including the amino acid sequence QAAKEFIAWLVK X³⁵R (SEQ ID NO.
   12), said residues of the sequence optionally including side chain protection; and
f) coupling the fifth fragment to the third fragment in order to provide an insulinotropic peptide including the amino acid sequence HX⁸EX¹⁰TFTSDVX¹⁷⁻¹⁸YLEGQAAKEFIAWLVK X³⁵R (SEQ ID NO. 13), said residues of the sequence optionally including side chain protection.

Preferably, the present invention relates to the method of making an insulinotropic peptide, comprising the steps of:
a) providing a first peptide fragment including the amino acid sequence HX⁸EX¹⁰ (SEQ ID NO. 6), wherein X⁸ and X¹⁰ are each residues of an achiral amino acid, each of H and E optionally including side chain protection;
b) providing a second peptide fragment including the amino acid sequence TFTSDVX¹⁷⁻¹⁸YLEG (SEQ ID NO. 8) wherein the residue denoted by the symbol X¹⁷⁻¹⁸ is a dipeptide residue of a pseudoproline, said amino acid residues of the sequence optionally including side chain protection;
c) coupling the first fragment to the second fragment to provide a third peptide fragment including the amino acid sequence HX⁸EX¹⁰TFTSDVX¹⁷⁻¹⁸YLEG (SEQ ID NO. 11), said amino acid residues of the sequence optionally including side chain protection;
d) providing a fourth peptide fragment including the amino acid sequence QAAKEFIAWLVKX³⁵ (SEQ ID NO. 9), wherein X³⁵ is a residue of an achiral amino acid, said amino acid residues of the sequence optionally including side chain protection;
e) coupling the fourth peptide fragment to arginine in order to provide a fifth peptide fragment including the amino acid sequence QAAKEFIAWLVK X³⁵R (SEQ ID NO.
   12), said residues of the sequence optionally including side chain protection; and
f) coupling the fifth fragment to the third fragment in order to provide an insulinotropic peptide including the amino acid sequence HX⁸EX¹⁰TFTSDVX¹⁷⁻¹⁸YLEGQAAKEFIAWLVK X³⁵R (SEQ ID NO. 13), said residues of the sequence optionally including side chain protection.

In another aspect, the invention relates to the method as described herein before, comprising a further step of:
g) removing the side chain protecting groups in order to provide an insulinotropic peptide including the amino acid sequence HX⁸EX¹⁰TFTSDVSSYLEGQAAKEFIAWLVKX³⁵R (SEQ ID NO. 5) and counterparts thereof, wherein each of the symbols X at positions, 8, 10, and 35 independently denotes an achiral, optionally sterically hindered amino acid residue.

Removing the side chain protecting groups is preferably carried out by employing a deprotection solution comprising at least one acidolytic reagent and at least one cation scavenger. Acidolytic reagents for global deprotection are preferably selected from the group consisting of trifluoroacetic acid (TFA), HCl, Lewis acids such as BF₃ Et₂O or Me₃SiBr, liquid hydrofluoric acid (HF), hydrogen bromide (HBr), trifluoromethanesulfonic acid, and combinations thereof. Suitable cation scavengers are preferably selected from dithiothreitol (DTT), anisole, p-cresol, ethanedithiol and dimethyl sulfide. The deprotection solution can also include water.

In another aspect, the present invention relates to a method of making an insulinotropic peptide, comprising the steps of:
a) providing a first peptide fragment including the amino acid sequence HX⁸EX¹⁰ (SEQ ID NO. 6), wherein X⁸ and X¹⁰ are each residues of an achiral amino acid, each of H, E, X⁸ and X¹⁰ optionally including side chain protection;
b) providing a second peptide fragment including the amino acid sequence TFTSDVX¹⁷⁻¹⁸YLEG (SEQ ID NO. 8) wherein the residue denoted by the symbol X¹⁷⁻¹⁸ is a dipeptide residue of a pseudoproline, said amino acid residues of the sequence optionally including side chain protection;
c) coupling the first fragment to the second fragment to provide a third peptide fragment including the amino acid sequence HX⁸EX¹⁰ TFTSDV X¹⁷⁻¹⁸YLEG (SEQ ID NO. 11), said amino acid residues of the sequence optionally including side chain protection;
d) providing a fourth peptide fragment including the amino acid sequence QAAKEFIAWLVKX³⁵ (SEQ ID NO. 9), wherein X³⁵ is a residue of an achiral amino acid, said amino acid residues of the sequence optionally including side chain protection;
e) coupling the fourth peptide fragment to arginine in order to provide a fifth peptide fragment including the amino acid sequence QAAKEFIAWLVK X³⁵R (SEQ ID NO.
   12), said residues of the sequence optionally including side chain protection; and
f) coupling the fifth fragment to the third fragment in order to provide an insulinotropic peptide of the formula (SEQ. ID No. 5) HX⁸EX¹⁰TFTSDVSSYLEGQAAKEFIAWLVKX³⁵R and counterparts thereof,
wherein each of the symbols X at positions, 8, 10, and 35 independently denotes an achiral, optionally sterically hindered amino acid residue; and wherein one or more of the amino acid residues optionally includes side chain protection.

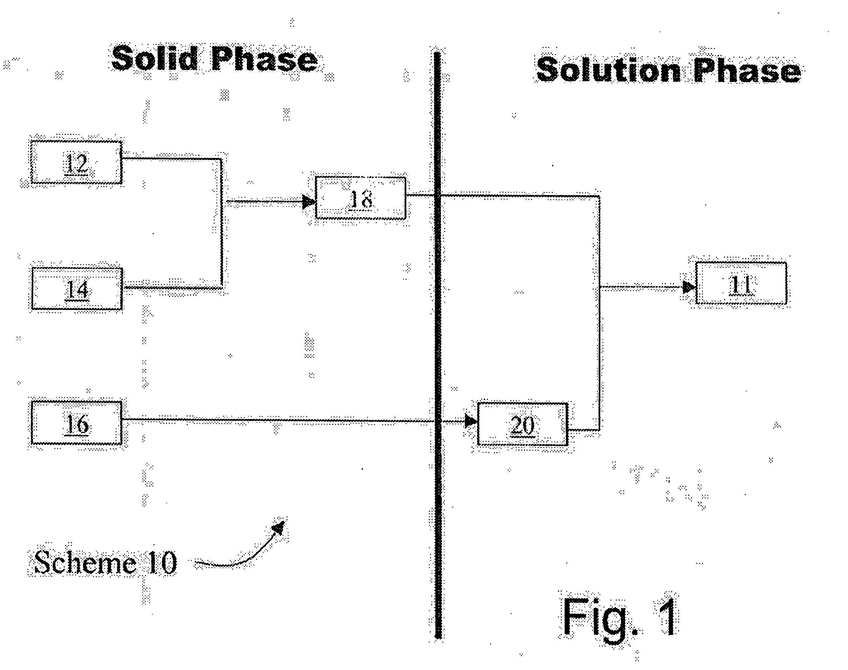

Fig. 1 is a schematic diagram of a synthesis scheme in accordance with the present invention.

Fragment 12 is a peptide fragment including the amino acid sequence HX⁸EX¹⁰ (SEQ ID NO. 6). Fragment 14 is a peptide fragment including the amino acid sequence T¹¹FTSD¹⁵VX¹⁷⁻¹⁸YL²⁰EG (SEQ ID NO. 8). Fragment 16 is a peptide fragment including the amino acid sequence Q²³AA²⁵KEFIA³⁰WLVKX³⁵ (SEQ ID NO. 9). Intermediate fragment 18 is a peptide fragment including the amino acid sequence H⁷X⁸EX¹⁰TFTSO¹⁵VX¹⁷⁻¹⁸YL²⁰EG (SEQ ID NO. 11). Intermediate peptide fragment 20 is a peptide including the amino acid sequence Q²³AA²⁵KEFIA³⁰WLVKX³⁵R (SEQ ID NO. 12). Product 11 is the desired protected peptide H⁷X⁸EX10TFTSD¹⁵VX¹⁷⁻¹⁸YL²⁰EG QAA²⁵KEFIA³⁰WLVKX³⁵R (SEQ ID NO. 13) in which the Ser-Ser at the 17 and 18 positions is still in the protected pseudoproline form. The diagram is described in more detail in the following.

The embodiments of the present invention described below are not intended to be exhaustive or to limit the invention to the precise forms disclosed in the following detailed description. Rather, the embodiments are chosen and described so that others skilled in the art can appreciate and understand the principles and practices of the present invention.

The present invention is directed to synthetic methods for making peptides such as the glucagon-like peptide-1 (GLP-1), and natural and non-natural insulinotropically active counterparts thereof, using solid and/or solution phase techniques. Peptide molecules of the invention may be protected, unprotected, or partially protected. Protection may include N-terminus protection, side chain protection, and/or C-terminus protection. While the invention is generally directed at the synthesis of these glucagon-like peptides, their counterparts, fragments and their counterparts, and fusion products and their counterparts of these, the inventive teachings herein can also be applicable to the synthesis of other peptides, particularly those that are synthesized using a combination of solid phase and solution phase approaches. The invention is also applicable to the synthesis of peptide intermediate fragments associated with impurities, particularly pyroglutamate impurities. Preferred GLP-1 molecules useful in the practice of the present invention include natural and non-natural GLP-1 (7-36) and counterparts thereof.

As used herein, the term "including the amino acid sequence" preferably means "having the amino acid sequence".

As used herein, a "counterpart" refers to natural and non-natural analogs, derivatives, fusion compounds, salts, or the like of a peptide. As used herein, a peptide analog generally refers to a peptide having a modified amino acid sequence such as by one or more amino acid substitutions, deletions, inversions, and/or additions relative to another peptide or peptide counterpart. Substitutions may involve one or more natural or non-natural amino acids. Substitutions preferably may be conservative or highly conservative. A conservative substitution refers to the substitution of an amino acid with another that has generally the same net electronic charge and generally the same size and shape. For instance, amino acids with aliphatic or substituted aliphatic amino acid side chains have approximately the same size when the total number of carbon and heteroatoms in their side chains differs by no more than about four. They have approximately the same shape when the number of branches in their side chains differs by no more than about one or two. Amino acids with phenyl or substituted phenyl groups in their side chains are considered to have about the same size and shape. Listed below are five groups of amino acids. Replacing an amino acid in a compound with another amino acid from the same groups generally results in a conservative substitution.
Group I: glycine, alanine, valine, leucine, isoleucine, serine, threonine, cysteine, methionine and non-naturally occurring amino acids with C₁-C₄ aliphatic or C₁-C₄ hydroxyl substituted aliphatic side chains (straight chained or monobranched).
Group II: glutamic acid, aspartic acid and nonnaturally occurring amino acids with carboxylic acid substituted C₁-C₄ aliphatic side chains (unbranched or one branch point).
Group III: lysine, ornithine, arginine and non-naturally occurring amino acids with amine or guanidino substituted C₁-C₄ aliphatic side chains (unbranched or one branch point).
Group IV: glutamine, asparagine and non-naturally occurring amino acids with amide substituted C₁-C₄ aliphatic side chains (unbranched or one branch point).
Group V: phenylalanine, phenylglycine, tyrosine and tryptophan.

As used herein, the term "counterpart" more preferably refers to the salts of a peptides, or to the derivatives thereof that are amidated at the C-terminus.

A "highly conservative substitution" is the replacement of an amino acid with another amino acid that has the same functional group in the side chain and nearly the same size and shape. Amino acids with aliphatic or substituted aliphatic amino acid side chains have nearly the same size when the total number carbon and heteroatoms in their side chains differs by no more than two. They have nearly the same shape when they have the same number of branches in the their side chains. Examples of highly conservative substitutions include valine for leucine, threonine for serine, aspartic acid for glutamic acid and phenylglycine for phenylalanine.

A "peptide derivative" generally refers to a peptide, a peptide analog, or other peptide counterpart having chemical modification of one or more of its side groups, alpha carbon atoms, terminal amino group, and/or terminal carboxyl acid group. By way of example, a chemical modification includes, but is not limited to, adding chemical moieties, creating new bonds, and/or removing chemical moieties. Modifications at amino acid side groups include, without limitation, acylation of lysine e-amino groups, N-alkylation of arginine, histidine, or lysine, alkylation of glutamic or aspartic carboxylic acid groups, and deamidation of glutamine or asparagine. Modifications of the terminal amino group include, without limitation, the des-amino, N-lower alkyl, N-di-lower alkyl, and N-acyl (e.g., -CO-lower alkyl) modifications. Modifications of the terminal carboxy group include, without limitation, the amide, lower alkyl amide, dialkyl amide, and lower alkyl ester modifications. Preferred derivatives are the derivatives are those which an amidated at the terminal carboxy group, e.g. the amide, lower alkyl amide or dialkyl amide of the peptide. Thus, partially or wholly protected peptides constitute peptide derivatives.

In the practice of the present invention, a compound has "insulinotropic" activity if it is able to stimulate, or cause the stimulation of, or help cause the stimulation of the synthesis or expression of the hormone insulin. In preferred modes of practice, insulinotropic activity can be demonstrated according to assays described in U.S. Pat. Nos. 6,887,849 and 6,703,365.

In preferred embodiments, the present invention provides methodologies for synthesizing synthetic (X⁸, X¹⁰, X³⁵)GLP-1 (7-36) peptides having the following formula (SEQ. ID NO. 5):
HX⁸EX¹⁰TFTSDVSSYLEGQAAKEFIAWLVKX³⁵R
   and counterparts thereof, wherein each of the symbols X at positions, 8, 10, and 35 independently denotes an achiral, optionally sterically hindered amino acid residue. Any of the X⁸, X¹⁰, and/or X³⁵ residues optionally may include side chain protecting group(s). Peptides according to this formula differ from the native GLP-1(7-36) at least in that the achiral, optionally sterically hindered X⁸ and X³⁵residues are substituted for the native amino acid residues at positions 8 and 35. The X¹⁰ residue may be derived from the native achiral glycine or another achiral amino acid. The use of the achiral X⁸, X¹⁰, and X³⁵amino acids not only help to stabilize the resultant peptide, but it has also now been discovered that the use of these amino acids as building blocks also facilitate the facile synthesis route of the present invention as shown in Fig. 1 and described further below.

A particularly preferred embodiment of a (X⁸, X¹⁰, X³⁵) GLP-11 (7-36) peptide that may be synthesized in accordance with principles of the present invention includes a peptide according to the formula (SEQ ID NO. 4):
HAibEGTFTSDVSSYLEGQAAKEFIAWLVKAibR
   and counterparts thereof, which preferably is amidated at the C-terminus. This peptide uses the achiral residue of alpha-aminoisobutyric acid (or methylalanine, shown schematically by the abbreviation Aib) as both X⁸ and X³⁵, preferably has an amide at the C-terminus, uses a residue of the native G at the 10 position, and may be designated by the formula (Aib ^{8,35})GLP-1(7-36)-NH₂. This notation indicates that an amino acid residue corresponding to the amino acid "Aib" appears at the 8 and 35 positions in place of the native alanine. The achiral alpha-aminoisobutric acid, also is known as methylalanine. The peptide according to SEQ ID NO. 4 is described in EP 1137667 B1. The presence of the Aib residues at the 8 and 35 positions slows metabolic degradation in the body, making this peptide much more stable in the body than the native GLP-1(7-36) peptide.

The present invention provides improved methodologies for making GLP-1(7-36) peptides such as the (Aib ^{8,35})GLP-1(7-36)-NH₂. By way of example, Fig. 1 schematically shows one illustrative scheme 10 for synthesizing GLP-1(7-36) peptides and their counterparts. The scheme 10 of Fig. 1 is believed to be particularly suitable for the scaled-up synthesis of GLP-1(7-36) peptides. Scaled-up procedures are typically performed to provide an amount of peptide useful for commercial distribution. For example the amount of peptide in a scaled-up procedure can be 500g, or 1 kg per batch, and more typically tens of kg to hundreds of kg per batch or more. In preferred embodiments, the inventive methods can provide such improvements as reduction in processing (synthesis) time, improvements in the yield of products, improvements in product purity, and/or reduction in amount of reagents and starting materials required.

The synthesis scheme 10 shown in Fig. 1 uses a combination of solid and solution phase techniques to prepare the peptide product 11.

As shown in Fig. 1, scheme 10 involves synthesizing peptide intermediate fragments 12, 14, and 16 on the solid phase. Fragment 12 is a peptide fragment including amino acid residues according to SEQ ID NO. 6:

HX⁸EX¹⁰

wherein X⁸ and X¹⁰ are as defined above, or is a counterpart thereof including the X⁸ and X¹⁰ residues. One or more of the amino acid residues may include side chain protecting groups in accordance with conventional practices. In some embodiments, the peptide fragment 12 may be resin bound via the C-terminus. This fragment optionally may bear N-terminus and/or C-terminus protection groups. Fmoc has been found to be a particularly useful N-terminus protecting group with respect to solid phase synthesis of the peptide fragment.

Fragment 12 includes the 4 amino acid residues corresponding to the amino acids in the 7 through 10 positions of the native GLP-1(7-36) peptide, and therefore may be represented by the notation (X⁸, X¹⁰)GLP-1 (7-10). In preferred embodiments, X⁸ is Aib and X¹⁰ is glycine according to SEQ ID NO. 7:

H⁷AibEG¹⁰

or is a counterpart thereof including the Aib residue at the 10 position. The peptide fragment according to SEQ ID NO. 7 may be represented by the notation (Aib⁸)GLP-1(7-10) to note the substitution of Aib for the native alanine at the 8 position of the native GLP-1(7-10).

Solid phase synthesis is generally carried out in a direction from the C-terminus to the N-terminus of the fragment 12. Thus, the X¹⁰ amino acid, which is present on the C-terminal portion of the fragment, is the first amino acid residue that is coupled to the solid phase resin support. Solid phase synthesis then proceeds by consecutively adding amino acid residues in a manner corresponding to the desired sequence. The synthesis of the peptide intermediate fragment is complete after the N-terminal residue (for example, the N-terminal histidine residue (H) has been added to the nascent peptide chain.

The selection and use of a peptide fragment according to SEQ ID NOS. 6 and 7 provides significant advantages within scheme 10. Firstly, H tends to be a difficult amino acid residue to add to a growing peptide chain due, at least in part, to epimerization issues. However, fragment 12 is small enough to alleviate these concerns in large part. Yet, fragment 12 is long enough to have two chiral centers. Thus, a simple crystallization allows the fragment to be purified. If fragment 12 ended at Aib, the fragment would have only one chiral center and would be, as a consequence, more difficult to purify racemically. Causing the achiral G to be positioned at the C-terminus also avoids racemization concerns that might otherwise be a concern if fragment 12 were to end at the C-terminus with the chiral E. In short, the selection of fragment 12 as a peptide building block makes it easier to build the fragment, purify it, and couple it to other peptide material. The fragment selection also enjoys low racemization of H. Surprisingly, H is added to this fragment with a very low level of epimerization, e.g., about 3% by weight in some modes of practice.

Fragment 14 is a peptide fragment including amino acid residues according to SEQ ID NO. 8:

T¹¹FTSD¹⁵VX¹⁷⁻¹⁸YL²⁰EG

wherein the residue denoted by the symbol X¹⁷⁻¹⁸ is a dipeptide residue of a pseudoproline, defined further below, or is a counterpart thereof including the X¹⁷⁻¹⁸ at the 17 and 18 positions. Fragment 14 includes amino acid residues generally corresponding to the amino acid residues in the 11 through 22 positions of the native GLP-1(7-36) peptide, except that the pseudoproline dipeptide residue X¹⁷⁻¹⁸is used instead of the SS (Ser-Ser) residues that occupy the corresponding 17 and 18 positions of the native GLP-1(7-36).

One or more of the amino acid residues of fragment 14 may include side chain protecting groups in accordance with conventional practices. In some embodiments, the peptide fragment 14 may be resin bound via the C-terminus. This fragment optionally may bear N-terminus and/or C-terminus protection groups. Fmoc has been found to be a particularly useful N-terminus protecting group with respect to solid phase synthesis of the peptide fragment. The peptide fragment according to SEQ ID NO. 8 may be referred to by the notation (X¹⁷⁻¹⁸)GLP-1(11-22) to note the substitution of the X¹⁷⁻¹⁸ pseudoproline residue for the Ser-Ser residue at the 17 and 18 positions.

As used in the practice of the present invention, the term pseudoproline refers to a dipeptide that includes a residue of a hydroxyl functional amino acid such as Ser or Thr in which the hydroxyl functional side chain is protected as a proline-like, TFA labile, oxazolidine ring between the alpha-amino and the side chain hydroxyl. As a consequence of the oxazolidine ring, the dipeptide functions as a reversible proline mimetic.

Generally, a typical pseudoproline residue as incorporated into a peptide may be represented by the formula 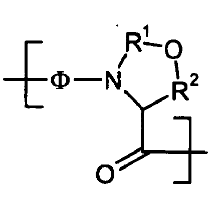 wherein Φ represents the residue of any amino acid and each of R¹ and R² is independently a suitable divalent linking moiety. Often, R¹ is a divalent moiety of the formula 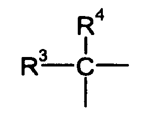 wherein each of R³ and R⁴ is independently a monovalent moiety such as H, or lower alkyl such as methyl. R³ and R⁴ also may be co-members of a ring structure. Desirably, each of R³ and R⁴ is methyl. In the case of an oxazolidinine ring-protected Ser, R² is the divalent moiety CH₂, while in the case of Thr, R² is the divalent moiety (CH₃)CH.

The term "lower alkyl" refers to a branched or straight-chain monovalent alkyl radical of one to six carbon atoms, preferably one to four carbon atoms. This term is further exemplified by radicals such as methyl, ethyl, n-propyl, isopropyl, n-butyl, s-butyl, isobutyl, t-butyl, n-pentyl, 3-methylbutyl, n-hexyl, 2-ethylbutyl and the like. Preferable lower alkyl residues are methyl and ethyl, with methyl being especially preferred.

During de-protection, the R¹ moiety is cleaved to provide a dipeptide residue according to the following formula: 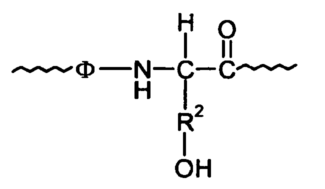 wherein Φ and R² are as defined above. As applied to fragment 14, the pseudoproline residue preferably corresponds to a Ser-Ser residue in which the Ser that is more proximal to the C-terminus is protected with the oxazolidine ring and has the following structure:

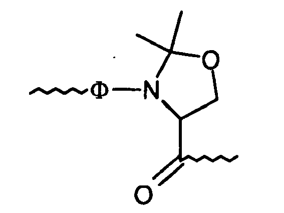

The hydroxyl-bearing side-chain of the Ser closer to the N-terminus is protected, such as by a t-Bu protection group. When the protecting oxazolidine ring structure and t-Bu are cleaved, the Ser-Ser residue results.

The use of such a proline mimetic as a building block in the synthesis of fragment 14 provides significant advantages in the context of the present invention. Firstly, the solid phase synthesis of fragment 14 is eased tremendously. When the pseudoproline is not used in the course of solid phase synthesis of fragment 14, there can be significant problems with Fmoc removals from residues 13 through 11. It is believed that this difficulty may be due to beta sheet formation. Use of the pseudoproline makes these Fmoc removals much easier by, it is believed, reducing the degree of beta sheet formation. Secondly, the subsequent solid phase coupling of fragment 14 to fragment 12 to form fragment 18, described below, is greatly eased. In the absence of the pseudoproline residue, the solubility of fragment 18 in typical solution phase coupling solvents is very poor. The pseudoproline enhances the solubility characteristics of the fragment 18, easing the solution phase coupling involving this fragment to fragment 20 (See discussion of Fig. 1, below).

Solid phase synthesis is generally carried out in a direction from the C-terminus to the N-terminus of the fragment 14. Thus, the G amino acid, which is present on the C-terminal portion of the fragment, is the first amino acid residue that is coupled to the solid phase resin support. Solid phase synthesis then proceeds by consecutively adding amino acid residues in a manner corresponding to the desired sequence. However, the X¹⁷⁻¹⁸pseudoproline dipeptide is added to the growing chain in a position corresponding to the 17 and 18 positions of GLP-1(7-36) instead of consecutively adding a pair of the native Ser residues at the 17 and 18 positions. The synthesis of the peptide intermediate fragment is complete after the N-terminal residue (for example, the N-terminal threonine residue (T) has been added to the nascent peptide chain.

Fragment 16 is a peptide fragment, or counterpart thereof, including amino acid residues according to SEQ ID NO. 9:

Q²³AA²⁵KEFIA³⁰WLVKX³⁵

wherein X³⁵ is as defined above, or is a counterpart thereof including the X³⁵ residue. One or more of the amino acid residues may include side chain protecting groups in accordance with conventional practices. Fragment 16 includes the amino acid residues corresponding to the amino acids in the 23 through 35 positions of the native GLP-1(7-36) peptide, except that X³⁵ is at the 35 position in place of the native amino acid at that position. Fragment 16 may be represented by the notation (X³⁵)GLP-1(23-35).

In some embodiments, the peptide fragment 16 may be resin bound via the C-terminus. This fragment optionally may bear side chain, N-terminus and/or C-terminus protection groups. Fmoc has been found to be a particularly useful N-terminus protecting group with respect to solid phase synthesis of the peptide fragment.

In preferred embodiments, X³⁵ is Aib according to SEQ ID NO. 10:

Q²³AA²⁵KEFIA³⁰WLVKAib³⁵

or a counterpart thereof including the Aib at the 35 position. The peptide fragment according to SEQ ID NO. 10 may be represented by the notation (Aib ³⁵)GLP-1(23-35) to note the substitution of Aib for the native amino acid at the 35 position of the native GLP-1(7-36).

Note that fragment 16 according to SEQ ID NOS. 9 and 10 does not yet include the R (arg) residue in the 36 position at the C terminus. The Arg is subsequently coupled to the C terminus of fragment 16 in the solution phase, preferably using Arg without side chain protection. This strategy provides significant advantages within scheme 10 of Fig. 1, because it avoids undesirable side reactions that tend to occur as a consequence of using protected Arg. For instance, upon de-protection of protected Arg, by-products of the deprotection may tend to react with other constituents of the peptide, e.g., tryptophan. This reduces the amount of desired peptide available in the crude for purification.

Solid phase synthesis is generally carried out in a direction from the C-terminus to the N-terminus of the fragment 16. Thus, the X³⁵ amino acid, which is present on the C-terminal portion of the fragment, is the first amino acid residue that is coupled to the solid phase resin support. Solid phase synthesis then proceeds by consecutively adding amino acid residues in a manner corresponding to the desired sequence. The synthesis of the peptide intermediate fragment is complete after the N-terminal residue (for example, the N-terminal glutamine residue (Q) has been added to the nascent peptide chain. Any of the amino acids used in the synthesis of fragment 16 may include side chain protection in accordance with conventional practices.

Due to steric hindrance proximal to the X³⁵-loaded support resin, the coupling of lysine (34) and valine (33) onto the growing peptide chain can be problematic. Even with an excess of amino acid, it is difficult to force these coupling reactions to completion. Solvent choice and/or end-capping can help to alleviate this problem. It has been found that the nature of the coupling solvent can impact the degree to which the coupling goes to completion. In one set of experiments, for example, coupling reactions were carried out in a 3:1 NMP/DCM, 1:1 NMP/DCM, 1:1 DMF/DCM, and 3:1 DMF/DCM. The ratios in these solvent combinations are on a volume basis. NMP refers to N-methyl pyrrolidone, DCM refers to dichloromethane, and DMF refers to dimethylformamide. It was found that the coupling reactions proceeded farther to completion when using 1:1 DMF/DCM.

End-capping after each of the lysine and valine couplings can also be used to prevent unreacted resin-supported material from proceeding in further coupling reactions. The end-capped material is more easily removed during purification if desired. Conventional end-capping techniques may be used.

Continuing to refer to Fig. 1, the fragments 12, 14, and 16, along with Arg, are assembled to complete the desired peptide 11. To accomplish this, fragment 12 is added to fragment 14 on the solid phase to produce larger, intermediate fragment 18 incorporating amino acid residues according to SEQ ID NO. 11:

H⁷X⁸EX¹⁰TFTSD¹⁵VX¹⁷⁻¹⁸YL²⁰EG

wherein X⁸, X¹⁰, and X¹⁷⁻¹⁸ are as defined above. In a preferred embodiment, X⁸ is Aib, X¹⁰ is the native G, and X¹⁷⁻¹⁸ is a pseudoproline dipeptide residue as defined above. This intermediate peptide fragment may be represented by the notation (X⁸, X¹⁰, X¹⁷⁻¹⁸) GLP-1(7-22).

Fig. 1 further shows that Arg is added to the C-terminus of fragment 16 in the solution phase to yield the larger intermediate peptide fragment 20 incorporating amino acid residues according to SEQ ID NO. 12:

Q²³AA²⁵KEFIA³⁰WLVKX³⁵R

wherein X³⁵ is as defined above and is preferably Aib. Preferably, the Arg added to the peptide fragment in this way does not include side chain protection. This intermediate peptide fragment 20 may be represented by the notation (X³⁵)GLP-1(23-36). Peptide fragments 18 and 20 are then coupled in the solution phase to yield the desired protected peptide 11 according to SEQ ID NO. 13 in which the Ser-Ser at the 17 and 18 positions is still in the protected pseudoproline form:

H⁷X⁸EX¹⁰TFTSD¹⁵VX¹⁷⁻¹⁸YL²⁰EG QAA²⁵KEFIA³⁰WLVKX³⁵R

The peptide 11 may be designated by the notation (X⁸, X¹⁰, X¹⁷⁻¹⁸, X³⁵)GLP-1(7-36). To the extent that the other amino acids bear side chain protection, this protection desirably is maintained through this step.

In carrying out the reaction scheme of Fig. 1, solid phase and solution phase syntheses may be carried out by standard methods known in the industry. In representative modes of practice, peptides are synthesized in the solid phase using chemistry by which amino acids are added from the C-terminus to the N-terminus.
Thus, the amino acid or peptide group proximal to the C-terminus of a particular fragment is the first to be added to the resin. This occurs by reacting the C-terminus functionality of the amino acid or peptide group with complementary functionality on the resin support. The N-terminus side of the amino acid or peptide group is masked to prevent undesired side reactions. The amino acid or peptide group desirably also includes side chain protection as well. Then successive amino acids or peptide groups are attached to the support-bound peptide material until the peptide of interest is formed. Most of these also include side chain protection in accordance with conventional practices. With each successive coupling, the masking group at the N-terminus end of the resin bound peptide material is removed. This is then reacted with the C-terminus of the next amino acid whose N-terminus is masked. The product of solid phase synthesis is thus a peptide bound to a resin support.

Any type of support suitable in the practice of solid phase peptide synthesis can be used. In preferred embodiments, the support comprises a resin that can be made from one or more polymers, copolymers or combinations of polymers such as polyamide, polysulfamide, substituted polyethylenes, polyethyleneglycol, phenolic resins, polysaccharides, or polystyrene. The polymer support can also be any solid that is sufficiently insoluble and inert to solvents used in peptide synthesis. The solid support typically includes a linking moiety to which the growing peptide is coupled during synthesis and which can be cleaved under desired conditions to release the peptide from the support. Suitable solid supports can have linkers that are photo-cleavable, TFA-cleavable, HF-cleavable, fluoride ion-cleavable, reductively-cleavable; Pd(O)-cleavable; nucleophilically-cleavable; or radically-cleavable. Preferred linking moieties are cleavable under conditions such that the side-chain groups of the cleaved peptide are still substantially globally protected.

In one preferred method of synthesis, the peptide intermediate fragments synthesized on an acid sensitive solid support that includes trityl groups, and more preferably on a resin that includes trityl groups having pendent chlorine groups, for example a 2-chlorotrityl chloride (2-CTC) resin (Barlos et al. (1989) Tetrahedron Letters 30(30):3943-3946). Examples also include trityl chloride resin, 4-methyltrityl chloride resin, 4-methoxytrityl chloride resin, 4-aminobutan-1-ol 2-chlorotrityl resin, 4-aminomethylbenzoyl 2-chlorotrityl resin, 3-aminopropan-1-ol 2-chlorotrityl resin, bromoacetic acid 2-chlorotrityl resin, cyanoacetic acid 2-chlorotrityl resin, 4-cyanobenzoic acid 2-chlorotrityl resin, glicinol 2-chlorotrityl resin, propionic 2-chlorotrityl resin, ethyleneglycol 2-chlorotrityl resin, N-Fmoc hydroxylamine 2-chlorotrityl resin, hydrazine 2-chlorotrityl resin. Some preferred solid supports include polystyrene, which can be copolymerized with divinylbenzene, to form support material to which the reactive groups are anchored.

Other resins that are used in solid phase synthesis include "Wang" resins, which comprise a copolymer of styrene and divinylbenzene with 4-hydroxymethylphenyloxymethyl anchoring groups (Wang, S.S. 1973, J. Am. Chem. Soc.), and 4-hydroxymethyl-3-methoxyphenoxybutyric acid resin (Richter et al. (1994), Tetrahedron Letters 35(27):4705-4706). The Wang, 2-chlorotrityl chloride, and 4-hydroxymethyl-3-methoxyphenoxy butyric acid resins can be purchased from, for example, Calbiochem-Novabiochem Corp., San Diego, California.

In order to prepare a resin for solid phase synthesis, the resin can be pre-washed in suitable solvent(s). For example, a solid phase resin such as a 2-CTC resin is added to a peptide chamber and pre-washed with a suitable solvent. The pre-wash solvent may be chosen based on the type of solvent (or mixture of solvents) that is used in the coupling reaction, or vice versa. Solvents that are suitable for washing, and also the subsequent coupling reaction include dichloromethane (DCM), dichloroethane (DCE), dimethylformamide (DMF), and the like, as well as mixtures of these reagents. Other useful solvents include DMSO, pyridine, chloroform, dioxane, tetrahydrofuran, ethyl acetate, N-methylpyrrolidone, and mixtures thereof. In some cases coupling can be performed in a binary solvent system, such as a mixture of DMF and DCM at a volume ratio in the range of 9:1 to 1:9, more commonly 4:1 to 1:4.

The syntheses of the present invention preferably are carried out in the presence of appropriate protecting groups unless otherwise noted. The nature and use of protecting groups is well known in the art. Generally, a suitable protecting group is any sort of group that that can help prevent the atom or moiety to which it is attached, e.g., oxygen or nitrogen, from participating in undesired reactions during processing and synthesis. Protecting groups include side chain protecting groups and amino- or N-terminal protecting groups. Protecting groups can also prevent reaction or bonding of carboxylic acids, thiols and the like.

A side chain protecting group refers to a chemical moiety coupled to the side chain (i.e., R group in the general amino acid formula H₂N-C(R)(H)-COOH) of an amino acid that helps to prevent a portion of the side chain from reacting with chemicals used in steps of peptide synthesis, processing, etc. The choice of a side chain-protecting group can depend on various factors, for example, type of synthesis performed, processing to which the peptide will be subjected, and the desired intermediate product or final product. The nature of the side chain protecting group also depends on the nature of the amino acid itself. Generally, a side chain protecting group is chosen that is not removed during deprotection of the α-amino groups during the solid phase synthesis. Therefore the α-amino protecting group and the side chain protecting group are typically not the same.

In some cases, and depending on the type of reagents used in solid phase synthesis and other peptide processing, an amino acid may not require the presence of a side-chain protecting group. Such amino acids typically do not include a reactive oxygen, nitrogen, or other reactive moiety in the side chain.

Examples of side chain protecting groups include acetyl (Ac), benzoyl (Bz), tert-butyl, triphenylmethyl (trityl), tetrahydropyranyl, benzyl ether(Bzl) and 2,6-dichlorobenzyl (DCB), t-butoxycarbonyl (Boc), nitro, p-toluenesulfonyl (Tos), adamantyloxycarbonyl, xanthyl (Xan), benzyl, 2,6-dichlorobenzyl, methyl, ethyl and t-butyl ester, benzyloxycarbonyl (Z), 2-chlorobenzyloxycarbonyl(2-Cl-Z), t-amyloxycarbonyl (Aoc), and aromatic or aliphatic urethan-type protecting groups, photolabile groups such as nitro veratryl oxycarbonyl (NVOC); and fluoride labile groups such as trimethylsilyl oxycarbonyl (TEOC).

Preferred side chain protecting groups for amino acids commonly used to synthesize GLP-1 peptides in the practice of the present invention are shown in the following Table A:

**Table A:**

| **Amino Acid** | **Side Chain Protecting group(s)** |
|---|---|
| Aib | None |
| Ala | None |
| Arg | None |
| Asp | t-butyl ester (OtBu) |
| Gln | trityl (trt) |
| Glu | OtBu |
| Gly | None |
| His | trityl (trt) |
| Ile | None |
| Leu | None |
| Lys | t-butyloxycarbonyl (Boc) |
| Phe | None |
| Ser | t-butyl (tBu) |
| X¹⁷⁻¹⁸ (corresponding to Ser-Ser) | oxazolidine ring between alpha nitrogen and OH of Ser closer to the C-terminus; tBu on other Ser |
| Thr | tBu |
| Trp | Boc |
| Tyr | tBu |
| Val | None |

An amino-terminal protecting group includes a chemical moiety coupled to the alpha amino group of an amino acid. Typically, the amino-terminal protecting group is removed in a deprotection reaction prior to the addition of the next amino acid to be added to the growing peptide chain, but can be maintained when the peptide is cleaved from the support. The choice of an amino terminal protecting group can depend on various factors, for example, type of synthesis performed and the desired intermediate product or final product.

Examples of amino-terminal protecting groups include (1) acyl-type protecting groups, such as formyl, acrylyl (Acr), benzoyl (Bz) and acetyl (Ac); (2) aromatic urethane-type protecting groups, such as benzyloxycarbonyl (Z) and substituted Z, such as p-chlorobenzyloxycarbonyl, p-nitrobenzyloxycarbonyl, p-bromobenzyloxycarbonyl, p-methoxybenzyloxycarbonyl; (3) aliphatic urethan protecting groups, such as t-butyloxycarbonyl (Boc), diisopropylmethoxycarbonyl, isopropyloxycarbonyl, ethoxycarbonyl, allyloxycarbonyl; (4) cycloalkyl urethan-type protecting groups, such as 9-fluorenyl-methyloxycarbonyl (Fmoc), cyclopentyloxycarbonyl, adamantyloxycarbonyl, and cyclohexyloxycarbonyl; and (5) thiourethan-type protecting groups, such as phenylthiocarbonyl. Preferred protecting groups include 9-fluorenyl-methyloxycarbonyl (Fmoc), 2-(4-biphenylyl)-propyl(2)oxycarbonyl (Bpoc), 2-phenylpropyl(2)-oxycarbonyl (Poc) and t-butyloxycarbonyl (Boc).

Fmoc or Fmoc-like chemistry is highly preferred for solid phase peptide synthesis, inasmuch as cleaving the resultant peptide in a protected state is relatively straightforward to carry out using mildly acidic cleaving agents. This kind of cleaving reaction is relatively clean in terms of resultant by-products, impurities, etc., making it technically and economically feasible to recover peptide on a large scale basis from both the swelling and shrinking washes, enhancing yield. As used herein, "large scale" with respect to peptide synthesis generally includes the synthesis of peptides in the range of at least 500 g, more preferably at least 2 kg per batch. Large-scale synthesis is typically performed in large reaction vessels, such as steel reaction vessels, that can accommodate quantities of reagents such as resins, solvents, amino acids, chemicals for coupling, and deprotection reactions, that are sized to allow for production of peptides in the kilogram to metric ton range.

Additionally, the Fmoc protecting group can be selectively cleaved from a peptide relative to the side chain protecting groups so that the side chain protection are left in place when the Fmoc is cleaved. This kind of selectivity is important during amino acid coupling to minimize side chain reactions. Additionally, the side chain protecting groups can be selectively cleaved to remove them relative to the Fmoc, leaving the Fmoc in place. This latter selectivity is very advantageously relied upon during purification schemes described further below.

The solid phase coupling reaction can be performed in the presence of one or more compounds that enhance or improve the coupling reaction. Compounds that can increase the rate of reaction and reduce the rate of side reactions include phosphonium and uronium salts that can, in the presence of a tertiary base, for example, diisopropylethylamine (DIEA) and triethylamine (TEA), convert protected amino acids into activated species (for example, BOP, PyBOPO, HBTU, and TBTU all generate HOBt esters). Other reagents help prevent racemization by providing a protecting reagent. These reagents include carbodiimides (for example, DCC or WSCDI) with an added auxiliary nucleophile (for example, 1-hydroxy-benzotriazole (HOBt), 1-hydroxyazabenzotriazole (HOAt), or HOSu). The mixed anhydride method, using isobutyl chloroformate, with or without an added auxiliary nucleophile, may also be utilized, as can the azide method, due to the low racemization associated with it. These types of compounds can also increase the rate of carbodiimide-mediated couplings, as well as prevent dehydration of Asn and Gln residues.

After the coupling is determined to be complete, the coupling reaction mixture is washed with a solvent, and the coupling cycle is repeated for each of the subsequent amino acid residues of the peptide material. In order to couple the next amino acid, removal of the N-terminal protecting group (for example, an Fmoc group) from the resin-bound material is typically accomplished by treatment with a reagent that includes 20-50% (on a weight basis) piperidine in a solvent, such as N-methylpyrrolidone (NMP) or dimethylformamide (DMF). After removal of the Fmoc protecting group, several washes are typically performed to remove residual piperidine and Fmoc by-products (such as dibenzofulvene and its piperidine adduct).

The subsequent amino acids can be utilized at a stoichiometric excess of amino acids in relation to the loading factor of peptide material on the resin support. Generally, the amount of amino acids used in the coupling step is at least equivalent to the loading factor of the first amino acid on the resin (1 equivalent or more). Preferably the amount of amino acids used in the coupling step is at least 1.3 equivalent (0.3 excess) or more, and most preferably about 1.5 equivalent (0.5 excess) or more. In some cases, for example, the coupling step utilizes an amount equivalent of amino acids in the range between 1 and 3.

Following the final coupling cycle, the resin is washed with a solvent such as NMP, and then washed with an inert second solvent such as DCM. In order to remove the synthesized peptide material from the resin, a cleaving treatment is carried out in a manner such that the cleaved peptide material still bears sufficient side chain and terminus protecting groups. Leaving the protective groups in place helps to prevent undesirable coupling or other undesirable reactions of peptide fragments during or after resin cleavage. In the case when Fmoc or similar chemistry is used to synthesize the peptide, protected cleavage may be accomplished in any desired fashion such as by using a relatively weak acid reagent such as acetic acid or dilute TFA in a solvent such as DCM. The use of 0.5 to 10 weight percent, preferably 1 to 3 weight percent TFA in DCM is typical. See, e.g., U.S. Pat. No. 6,281,335.

Steps of cleaving the peptide intermediate fragment from the solid phase resin can proceed along the lines of an exemplary process as follows. However, any suitable process that effectively cleaves the peptide intermediate fragment from the resin can be used. For example, approximately 5 to 20, preferably about 10 volumes of a solvent containing an acidic cleaving reagent is added to the vessel containing the resin-bound peptide material, The resin, typically in the form of beads, is immersed in the reagent as a consequence. The cleaving reaction occurs as the liquid contents are agitated at a suitable temperature for a suitable time period. Agitation helps prevent the beads from clumping. Suitable time and temperature conditions will depend upon factors such as the acid reagent being used, the nature of the peptide, the nature of the resin, and the like. As general guidelines, stirring at from about -15°C to about 5°C, preferably from about -10°C to about 0°C for about 5 minutes to two hours, preferably about 25 minutes to about 45 minutes would be suitable. Cleaving time may be in the range of from about 10 minutes to about 2 hours or even as much as a day. Cleaving is desirably carried out in such a chilled temperature range to accommodate a reaction exotherm that might typically occur during the reaction. In addition, the lower temperature of the cleavage reaction prevents acid sensitive side chain protecting groups, such as trityl groups, from being removed at this stage.

At the end of the cleaving treatment, the reaction is quenched. This may be achieved, for example, by combining the cleaving reagent with a suitable base, such as pyridine or the like, and continuing to agitate and stir for an additional period such as for an additional 5 minutes to 2 hours, preferably about 20 minutes to about 40 minutes. Adding the base and continued agitation causes the temperature of the vessel contents to increase. At the end of agitation, the vessel contents may be at a temperature in the range of from about 0 °C to about 15 °C, preferably about 5 °C to about 10 °C.

Factors such as swelling and shrinking the resin in order to improve aspects of the peptide recovery can optionally be incorporated into the overall synthesis process. These techniques are described, for example, in U.S. Pat. Pub. No. 2005/0164912 A1.

In some aspects, the cleaved peptide fragments can be prepared for solution phase coupling to other peptide fragments and/or amino acids. Peptide coupling reactions in the solution phase are reviewed in, for example, New Trends in Peptide Coupling Reagents; Albericio, Fernando; Chinchilla, Rafeal; Dodsworth, David J.; and Najera, Armen; Organic Preparations and Procedures International (2003), 33(3), 203-303.

Coupling of peptide intermediate fragments to other fragments or amino acid(s) in the solution phase can be carried out using *in situ* coupling reagents, for example 2-(1H-benzotriazol-1-yl)tris(dimethylainino)phosphonium hexafluorophosphate (BOP), o-(benzotriazol-1-yl)-N,N,N',N'-tetramethyluronium hexafluorophosphate (HBTU), o-(7-azobenzotriazol-1-yl)-1,1,3,3-tetramethyluronium hexafluorophosphate (HATU), dicyclohexylcarbodiimide (DCC), water-soluble carbodiimide (WSCDI), or o-(benzotriazol-1-yl)-N,N,N',N'-tetramethyluronium tetrafluoroborate (TBTU). Other coupling techniques use preformed active esters such as hydroxysuccinimide (HOSu) and p-nitrophenol (HONp) esters; preformed symmetrical anhydrides; non-symmetrical anhydrides such as N-carboxyanhydrides (NCAs); or acid halides such as acyl fluoride as well as acyl chloride.

A suitable coupling solvent can be used in the solution phase coupling reaction. It is understood that the coupling solvent(s) used can affect the degree of racemization of the peptide bond formed; the solubility of the peptide and/or peptide fragments; and the coupling reaction rate. In some embodiments, the coupling solvent includes one or more water-miscible reagents. Examples of water-miscible solvents include, for example, DMSO, pyridine, chloroform, dioxane, tetrahydrofuran, ethyl acetate, N-methylpyrrolidone, dimethylformamide, dioxane, or mixtures thereof.

In other embodiments, the coupling reaction may include one or more non water-miscible reagents. An exemplary non water-miscible solvent is methylene chloride. In these embodiments, the non water-miscible solvent is preferably compatible with the deprotection reaction; for example, if a non water-miscible solvent is used preferably it does not adversely affect the deprotection reaction.

After the peptide 11 is formed, the product can be subject to deprotection, purification, lyophilization, further processing (e.g., reaction with another peptide to form a fusion protein); combinations of these, and/or the like, as desired.

For example, according to the invention, the side-chain protecting groups are typically retained on the peptide intermediate fragments throughout solid phase synthesis and also into and throughout the solution phase coupling reactions. Generally, after solution phase coupling step is completed, one or more deprotection steps may be performed to remove one or more protecting groups from the peptide.

The removal of side chain protecting groups by global deprotection typically utilizes a deprotection solution that includes an acidolytic agent to cleave the side chain protecting groups. Commonly used acidolytic reagents for global deprotection include neat trifluoroacetic acid (TFA), HCl, Lewis acids such as BF₃Et₂O or Me₃SiBr, liquid hydrofluoric acid (HF), hydrogen bromide (HBr), trifluoromethanesulfonic acid, and combinations thereof. The deprotection solution also includes one or more suitable cation scavengers, for example, dithiothreitol (DTT), anisole, p-cresol, ethanedithiol, or dimethyl sulfide. The deprotection solution can also include water. As used herein, amounts of reagents present in the deprotection composition are typically expressed in a ratio, wherein the amount of an individual component is expressed as a numerator in "parts", such as "parts weight" or "parts volume" and the denominator is the total parts in the composition. For example, a deprotection solution containing TFA:H₂O:DTT in a ratio of 90:5:5 (weight/weight/weight) has TFA at 90/100 parts by weight, H₂O at 5/100 parts by weight, and DTT at 5/ 100 parts by weight.

In some embodiments, the deprotection reaction can be performed wherein the amount of the acidolytic agent, preferably TFA, in the deprotection composition is greater than 90/100 parts by weight. Other preferred deprotection compositions include an amount of acidolytic agent in an amount of 93/100 parts by weight or greater, or in an amount in the range of 93/100 by weight to 95/100 parts by weight.

The precipitation is typically done using an ether, e.g., diethyl ether or MTBE (Methyl Tert Butyl Ether). After precipitation, the peptide is desirably isolated and dried before being combined with other ingredients, lyophilized, packaged, stored, further processed, and/or otherwise handled. This may be accomplished in any suitable fashion. According to one suitable approach, the peptide is collected via filtering, washed with ample MTBE washes to reduce final salt content to a suitable level, and then dried.

The present invention also provides useful techniques for purifying a wide range of peptides, including GLP-1 peptides and their counterparts.

A particularly preferred purification process involves at least two purification passes through chromatographic media, wherein at least a first pass occurs at a first pH and at least a second pass occurs at a second pH. More preferably, the first pass occurs at an acidic pH, while the second pass occurs at a basic pH. In preferred embodiments, at least one pass under acidic conditions occurs prior to a pass occurring under basic conditions. An illustrative mode of practicing this purification approach can be described in the illustrative context of purifying fully protected peptide 11 resulting from the scheme 10 shown in Fig. 1. Initially, the peptide is globally de-protected. Both N-terminus and side chain protecting groups are cleaved. A first chromatographic pass is carried out in a water/ACN (acetonitrile) gradient, using enough TFA to provide a pH of about 1 to 5, preferably about 2. A second pass is then carried out in a water/ACN gradient using a little ammonia and/or ammonium acetate, or the like, to provide a pH of around 8 to 9, preferably 8.5 to 8.9.

The pH values, whether acid or base, promote uniformity in that a uniform ionic species is present in each instance. Thus, the acidic pH desirably is sufficiently low so that substantially all of the amino acid residues in the peptide material are protonated. The basic pH is desirably high enough so that substantially all of the amino acid residues in the peptide material are deprotonated. The acid and base chromatography can be carried out in any order. It is convenient to do the basic chromatography last when the peptide acetate is a desired product inasmuch as the acetate may be the product of chromatography.

The principles of the present invention will now be further illustrated with respect to the following illustrative examples. In the following all percentages and ratios are by volume unless otherwise expressly stated.

### Example 1 - Solid Phase Synthesis of Fragment 12 with Fmoc protection at the N-terminus, and side chain protection on the His and Glu.

### A. Preparation of Fmoc-Gly-loaded 2CTC Resin

Initially, Fmoc-Gly-loaded 2CTC resin was prepared. Amounts of reagents used are listed in following table:

| Preparation of Fmoc-Gly-2-Chlorotrityl Resin | | | | | |
|---|---|---|---|---|---|
| Materials | MW | Eq | mmol | grams | mL |
| 2-Chlorotritylchloride resin | - | - | 52.24 | 35.06 | - |
| Fmoc-Gly-OH | 297.3 | 1.0 | 13.06 | 3.88 | - |
| Diisopropylethylamine (DIEA) | 129.25 | 2.35 | 30.72 | 3.97 | |
| Dimethyl formamide (DMF) | | | | | 1270 |
| Dichloromethane (DCM) | | | | | 1785 |
| 9:1 by volume Methanol: DIEA | | | | | 350 |
| Isopropanol (IPA) | | | | | 1050 |

2-CTC resin was charged to a 500 mL peptide reactor and swelled with 400 mL DCM for 30 min at 25 °C. The bed was drained and a solution of Fmoc-Gly-OH and DIEA in 8 volume of DMF:DCM (87.5:12.5) was added. The mixture was stirred under nitrogen for 2 hours at a temperature of 25°C.

The bed was drained and washed once with 350 mL DMF and once with 175 mL DMF. Then, remaining active sites on the 2-CTC resin were end-capped with 350 mL of MeOH:DIEA (9:1) solution for 1 hour. The bed was drained, washed with 250 mL DMF two times, and then with 350 mL DCM four times. The resin was de-swelled by washing with 3X350 mL IPA. The resin was dried to a constant weight to give 38.20 g of loaded resin. Analysis showed a loading factor of 0.18 mmol/g.

### B. Solid Phase Synthesis

Solid phase synthesis was carried out starting with 20.0g of Fmoc-Gly-2-CTC resin loaded at 0.18 mmol/g as prepared in Part A of this Example 1. The resin was swelled in DCM (200 mL) for 30 min at 25 °C. The DCM solvent was drained and the resin was washed three times with NMP (5 vol. each wash).

The resin was then treated twice with 20% by volume piperidine in NMP (5 vol. each treatment) to remove Fmoc protecting groups. After the second 20% piperidine/NMP treatment, the resin was washed five times with NMP (5 vol. each wash) to a negative chloranil test.

To prepare the coupling solution, the amino acid (2.85 equiv.) and 6-Chloro-1-Hydroxybenzotriazole (6-Cl-HOBT, 2.85 equiv.) were weighed, dissolved in 2.55x volume of NMP then combined with DIEA (3.25 equiv.) at 5 °C to 10 °C. TBTU (2.85 equiv.) was dissolved in 1.3x volume of NMP at 5 °C to 10 °C. The two solutions were then combined. The resultant solution was added to the reaction vessel. The flask was rinsed with 1.3x volume of DCM added into the reactor, which was then stirred for 2-3 hours at 25 °C to 27 °C. The sample was pulled for Kaiser Test to check the reaction for completion. If the coupling reaction was incomplete after 3 hours (positive Kaiser Test), the reaction vessel was drained and recoupling was performed with fresh solution of activated amino acid. After completion of the coupling reaction, the coupling solution was drained and the resin was washed with NMP 4 times (5 vol. each wash). Then removal of the Fmoc protecting group and coupling reaction cycle was repeated for the remaining amino acids in the fragment (i.e., in the order of Glu(OtBu)→Aib-His(trt)).

Due to difficulty of the coupling reaction between activated Fmoc-His(trt)-OH and H-Aib-Glu(OtBu)-Gly-2-CTC and the instability of the activated Fmoc-His(trt)-OH, the coupling reaction was forced to completion by draining the reaction solution after one hour and immediately performing the recoupling reaction with a second, fresh activated Fmoc-His(trt)-OH solution.

All reagents used in Part B of this example are listed in following table:

| Amino Acid | g | 6-Cl-HOBT (g) | DIEA (g) | NMP (mL) | TBTU (g) | NMP (mL) | DCM (mL) | Coupling time (min) |
|---|---|---|---|---|---|---|---|---|
| Glu(OtBu) | 4.34 | 1.76 | 1.55 | 51.0 | 3.28 | 26.0 | 26.0 | 150 |
| Aib | 3.36 | 1.76 | 1.51 | 51.0 | 3.29 | 26.0 | 26.0 | 155 |
| His(trt) | 6.32 | 1.78 | 1.56 | 51.0 | 3.29 | 26.0 | 26.0 | 60 |
| His(trt) recoupling | 6.32 | 1.79 | 1.56 | 51.0 | 3.29 | 26.0 | 26.0 | 92 |

The resin-bound peptide fragment was washed with NMP (5 vol.) 4 times, DCM (6 vol.) 5 times, IPA (5 vol.) 3 times. The de-swelled resin was then dried at 35°C under vacuum to give 22.58 g resin and resin-bound peptide.

### C. Cleavage of the Fmoc and side-chain protected fragment from the resin

The built resin from Part B above was swelled in DCM (12.5 volumes relative to the weight of resin used; 12.5 ml DCM per g of resin or 12.5 liters per kg) for 30 min at 25 °C and then washed with DCM 2 times (6.25 vol. each wash) to remove any NMP residue. The resin was cooled with the last DCM wash to -5 °C. The DCM was drained and a cold solution of 1% TFA/DCM (10 vol. at -5 °C to -10 °C) was added and stirred for 30 min at 0 °C. Pyridine (1.3 equiv. of TFA) was added to the reactor to neutralize TFA. The cleavage solution was filtered off and collected in a flask. While the vessel warmed up to 25 °C, the resin was washed with DCM 7 times (7.5 vol.). The washes were combined with the cleavage solution. The DCM cleavage solution was combined with water (7.5 vol.). The resultant mixture was distilled under reduced pressure to remove DCM (350 torr at 28 °C). The peptide fragment precipitated out from the water when DCM was removed. The fragment was washed with water and dried at 30 °C-35 °C under vacuum. A total of 4.73 g of Fmoc-(Aib⁸)GLP-1(7-10)-OH was obtained.

### Example 2

### A. Preparation of Fmoc-Gly-loaded 2CTC Resin

Fmoc-Gly-loaded 2CTC resin was prepared. The amounts of reagents used are listed in following table:

| Preparation of Fmoc-Gly-2-Chlorotrityl Resin | | | | | |
|---|---|---|---|---|---|
| Materials | MW | Eq | mmol | grams | mL |
| 2-Chlorotritylchloride resin | - | - | 59.66 | 40.04 | - |
| Fmoc-Gly-OH | 297.3 | 1.0 | 29.84 | 8.87 | - |
| Diisopropylethylamine (DIEA) | 129.25 | 1.67 | 49.90 | 6.45 | |
| Dimethyl formamide (DMF) | | | | | 1580 |
| Dichloromethane (DCM) | | | | | 1840 |
| 9:1 Methanol: DIEA | | | | | 390 |
| Isopropanol (IPA) | | | | | 1050 |

2-CTC resin was charged to a 500-mL peptide reactor and swelled with 400 mL DCM for 30 min. The resin was drained, and a solution Fmoc-Gly-OH and DIEA in 8 volume of DMF:DCM (87.5:12.5 by volume) was added. The mixture was stirred under nitrogen for 2 hours at a temperature of 25 °C.

The resin bed was drained and washed once with 400 mL DMF and once with 200 mL DMF. Then, remaining active sites on the 2-CTC resin were end-capped with 390 mL of MeOH:DIEA (9:1 by volume) solution for 1 hour. The bed was drained again, washed two times with 350 mL DMF, and washed four times with 350 mL DCM. The resin was then de-swelled by washing with 3X350 mL IPA. The resin was dried at 35 °C under vacuum to a constant weight to give 48.51 g of loaded resin. Analysis showed a loading factor of 0.54 mmol/g.

### B. Solid Phase Synthesis

Solid phase synthesis was carried out starting with 27.59g of Fmoc-Gly-2-CTC resin loaded at 0.54 mmol/g. The resin was swelled in DCM (300 mL) for 30 min at 25 °C. The DCM solvent was drained, and the resin was washed and three times with NMP (5 vol. each wash).

The resin was then treated twice with 20% by volume piperidine in NMP (5 vol. each treatment) to remove Fmoc protecting groups. After the second 20% piperidine/NMP treatment, the resin was washed six times with NMP (5 vol. each wash) to a negative chloranil test.

To prepare the coupling solution, the amino acid (1.7 equiv.) and 6-Chloro-1-Hydroxybenzotriazole (6-Cl-HOBT, 1.7 equiv.) were weighed, dissolved in 4.6x volume of NMP, and then combined with DIEA (1.9 equiv.) at 5 °C to 10 °C. TBTU (1.7 equiv.) was dissolved in 2.28x volume of NMP at 5 °C to 10 °C. The two solutions were then combined. The resultant solution was added to the reaction vessel. The flask was rinsed with 2.28 volumes of DCM into the reactor, which was then stirred for 2-3 hours at 25 °C - 27 °C. The sample was pulled for a Kaiser Test to check the reaction for completion. After completion of the coupling reaction, the coupling solution was drained, and the resin was washed with NMP 4 times (5 vol. each wash). Removal of the Fmoc group and coupling reaction cycle was repeated for the remaining amino acids in the fragment (i.e., in the order of Glu(OtBu)→Aib-→His(trt)).

All reagents used in this example are listed in following table:

| Amino Acid | g | 6-Cl-HOBT (g) | DIEA (g) | NMP (mL) | TBTU (g) | NMP (mL) | DCM (mL) | Coupling time (min) |
|---|---|---|---|---|---|---|---|---|
| Glu(OtBu) | 10.79 | 4.24 | 2.67 | 127 | 8.13 | 63 | 63 | 156 |
| Aib | 8.26 | 4.32 | 3.73 | 125 | 8.14 | 65 | 65 | 180 |
| His(trt) | 15.68 | 4.31 | 3.69 | 125 | 8.12 | 65 | 65 | 180 |

### C. Cleavage of the fragment from the resin

The built resin was washed with NMP (5 vol.) 6 times and then DCM (6 vol.) 8 times to remove NMP residue. The resin was cooled with the last DCM wash to -5 °C. After draining DCM, a cold (-5 °C to -10 °C) solution of 1% TFA/DCM (10 vol.) was added, and the resultant pot mixture was stirred for 30 min at 0 °C. Pyridine (1.3 equiv., of TFA) was charged to the reactor to neutralize the TFA. The cleavage solution was collected in the flask. While the vessel warmed up to 25 °C, the resin was washed with DCM (7.5 vol.) 11 times and drained into the cleavage solution. The DCM solution was combined with water (10 vol.). The resultant mixture was distilled under reduced pressure to remove DCM (350 torr at 28 °C). The fragment precipitated out from water when DCM was removed. The fragment was washed with water and dried at 30 °C- 35 °C under vacuum. A total of 11.12 g Fmoc-(Aib⁸)GLP-1(7-10)-OH (78.8% yield) was obtained.

### Example 3

### A. Preparation of Fmoc-Gly-loaded 2CTC Resin

Fmoc-Gly-loaded 2CTC resin was prepared. The amounts of reagents used are listed in following table:

| Preparation of Fmoc-Gly-2-Chlorotrityl Resin | | | | | |
|---|---|---|---|---|---|
| Materials | MW | Eq | mmol | grams | mL |
| 2-Chlorotritylchloride resin | - | - | 60.88 | 40.86 | - |
| Fmoc-Gly-OH | 297.3 | 1.0 | 42.58 | 12.66 | - |
| Diisopropylethylamine (DIEA) | 129.25 | 1.48 | 63.21 | 8.17 | |
| Dimethyl formamide (DMF) | | | | | 1380 |
| Dichloromethane (DCM) | | | | | 1840 |
| 9:1 Methanol: DIEA | | | | | 390 |
| Isopropanol (IPA) | | | | | 1000 |

2-CTC resin was charged to a 500-mL peptide reactor and swelled with 400 mL DCM for 30 min. The bed was drained, and a solution Fmoc-Gly-OH and DIEA in 8 volume of DMF:DCM (87.5:12.5) was added. The mixture was stirred under nitrogen for 2 hours at a temperature of 25°C.

The bed was drained and washed once with 400 mL DMF. Then, any remaining active sites on the 2-CTC resin were end-capped with 390 mL of MeOH:DIEA (9:1) solution for 1 hour. The bed was drained, washed two times with 350 mL DMF, and then four times with 350 mL DCM. The resin was de-swelled by washing with 4 x 250 mL IPA. The resin was dried at 35 °C under vacuum to a constant weight to give 52.02 g of loaded resin. Analysis showed a loading factor of 0.72 mmol/g.

### B. Solid Phase Synthesis

Solid phase synthesis was carried out starting with 24.43g of Fmoc-Gly-2-CTC resin loaded at 0.72 mmol/g. The resin was swelled in DCM (250 mL) for 30 min at 25 °C. The DCM solvent was drained and the resin was washed and three times with NMP (5 vol. each wash).

The resin was then treated twice with 20% piperidine in NMP (5 vol. each treatment) to remove Fmoc protecting groups. After the second 20% piperidine/NMP treatment, the resin was washed six times with NMP (5 vol. each wash) to a negative chloranil test.

To prepare the coupling solution, the amino acid and 6-chloro-1-Hydroxybenzotriazole (6-Cl-HOBT) were weighed, dissolved in NMP and then combined with DIEA at 10 °C- 5 °C. TBTU was dissolved in NMP at 10 °C- 5 °C. The two solutions were then combined. The resultant solution was added to a reaction vessel. The flask was rinsed with DCM (see following table for amounts) into the reactor, which was stirred for 2-6 hours at 25 °C- 27 °C. The sample was pulled for Kaiser Test to check the reaction for completion. If the coupling reaction was incomplete after 3 hours (positive Kaiser Test), the reaction vessel was drained and recoupling was performed with fresh solution of activated amino acid. After the coupling reaction was completed, the coupling solution was drained and the resin was washed with NMP 4 times (5 vol. each wash). Then, removal of the Fmoc group and the coupling reaction cycle was repeated for the remaining amino acids in the fragment (i.e., in the order of Glu(OtBu)→Aib→His(trt)).

All reagents used in this example are listed in following table:

| Amino Acid | g / Eq | 6-Cl-HOBT (g/Eq) | DIEA (g/Eq) | NMP (mL) | TBTU (g/Eq) | NMP (mL) | DCM (mL) | Coupling time (min) |
|---|---|---|---|---|---|---|---|---|
| Glu(OtBu) | 12.40 | 4.95 | 4.29 | 145 | 9.37 | 70 | 70 | 180 |
| | /1.65 | /1.65 | /1.85 | | /1.65 | | | |
| Aib | 9.48 | 4.96 | 4.23 | 140 | 9.33 | | 70 | 352 |
| | /1.65 | /1.65 | /1.85 | | /1.65 | | | |
| Aib recoupling | 4.73 | 2.48 | 2.15 | 72 | 4.85 | 36 | 36 | 120 |
| | /0.83 | /0.83 | /0.92 | | /0.83 | | | |
| His(trt) | 21.18 | 5.80 | 4.99 | 140 | 10.98 | 70 | 70 | 180 |
| | /1.94 | /1.94 | /2.14 | | /1.94 | | | |
| His(trt) recoupling | 10.80 | 2.90 | 2.48 | 72 | 5.49 | 36 | 36 | 180 |
| | /0.97 | /0.97 | /1.07 | | /0.97 | | | |

### C. Cleavage of the fragment Fmoc-AA(7-10)-OH from the resin

The built resin was washed with NMP (5 vol.) 6 times and DCM (6 vol.) 7 times to remove NMP. The resin was cooled with the last DCM wash to -5 °C. The DCM was drained, and the resin bed was washed with a cold (-5 °C to -10 °C) solution of 1% TFA/DCM (11.26 vol.) for 5 min at 0 °C. The cleavage solution was collected in the flask, to which had been added pyridine (1.3 equiv. of total TFA) for neutralizing TFA. Then, the second portion of cold 1% TFA/DCM (6.14 vol.) was added to the reactor and stirred for 2 min. The second cleavage solution was again drained into the collecting flask. While the vessel warmed up to 25 °C, the resin was washed with DCM 9 times (8.2 vol.) and drained into the cleavage solution. The DCM solution was combined with water (8.2 vol.). The resultant mixture was distilled under reduced pressure to remove DCM (350 torr at 28 °C). The fragment precipitated out from water when DCM was removed. The fragment was washed with water and dried at 30 °C to 35 °C under vacuum. A total of 14.02 g of Fmoc-(Aib⁸)GLP-1(7-10)-OH (86.6% yield) according to SEQ ID NO. 7 was obtained. Analysis showed a purity of 94.3% AN.

### Example 4 - Solid Phase Synthesis of side chain protectedFmoc-(Aib³⁵) GLP-1 (23-35)-OH

### A. Preparation of Fmoc-Aib-loaded 2CTC Resin

Fmoc-Aib-loaded 2CTC resin was prepared. The amounts of reagents used are listed in following table:

| Preparation of Fmoc-Aib-2-Chlorotrityl Resin | | | | | |
|---|---|---|---|---|---|
| Materials | MW | Eq | mmol | grams | mL |
| 2-Chlorotritylchloride resin | - | - | 59.66 | 40.04 | - |
| Fmoc-Aib-OH | 325.5 | 1.0 | 14.91 | 4.85 | - |
| Diisopropylethylamine (DIEA) | 129.25 | 2.39 | 35.20 | 4.61 | |
| Dimethyl formamide (DMF) | | | | | 1480 |
| Dichloromethane (DCM) | | | | | 1840 |
| 9:1 Methanol: DIEA | | | | | 450 |
| Isopropanol (IPA) | | | | | 1050 |

2-CTC resin was charged to a 500 mL peptide reactor and swelled with 400 mL DCM for 30 min. The bed was drained, and a solution of Fmoc-Aib-OH and DIEA in 8 volume of DMF:DCM (87.5:12.5) was added. The mixture was stirred under nitrogen for 2 hours at a temperature of 25 °C.

The bed was drained and washed with DMF, 400 mL once and 200 mL a second time. Then, any remaining active sites on the 2-CTC resin were end-capped with 400 mL of MeOH:DIEA (9:1) solution for 1 hour. The bed was drained. The resin was washed once with 450 mL DMF/MeOH/DIEA (4:0.9:0.1), once with 200 mL DMF, and four times with 350 mL DCM. The resin was de-swelled by washing with 3 x 350 mL IPA. The resin was dried to a constant weight to give 45.15 g of loaded resin. Analysis showed a loading factor of 0.24 mmol/g.

### B. Solid Phase Synthesis

10.01 g of Fmoc-Aib-2-CTC resin with loading factor at 0.24 mmal/g were charged to a reaction vessel and swelled in DCM (120 mL) for 30 min at 25 °C. The DCM solvent was drained, and the resin was washed three times with NMP (6 vol. each wash).

The resin was then treated twice with 5% by volume piperidine in NMP (6 vol. each treatment) to remove Fmoc protecting groups. After the second 5% piperidine/NMP treatment, the resin was washed four times with NMP (6 vol. each wash).

To prepare the coupling solution, the amino acid (1.875 equiv.) and 1-hydroxy-benzotriazole monohydate (HOBT hydrate, 2.07 equiv.) were dissolved in 3.5x volume of NMP at 5 °C to 10 °C and then combined with a 16.1 mL solution of HBTU (2.0 equiv.) in NMP (1.5x vol.). Then 2.2 mL DIEA (2.63 equiv.) was added to the activation vessel at 10 °C- 5 °C. The resultant solution was transferred to a reaction vessel. The activation vessel was rinsed with 1.5x volume of DCM into the reactor, which was then stirred for 2 hours at 25 °C. The reaction vessel was drained. The coupling reaction was repeated one more time with fresh solution of activated amino acid (1.875 eq) After the second coupling reaction was completed, the coupling solution was drained and the resin was washed with NMP 4 times (6 vol. each wash). Then, removal of the Fmoc group and coupling reaction cycle was repeated for the remaining amino acids in the fragment (i.e., in the order of Lys(Boc)→Val→Leu→Trp(Boc)→Ala→Ile→Phe→Glu(OtBu)→ Lys(Boc)→Ala→Ala→Gln(trt)).

All reagents used in this example are listed in following table:

| Coupling Reaction of Fmoc-AA(23-35)-OH Example 1 | | | | | | | | |
|---|---|---|---|---|---|---|---|---|
| Amino Acid | g | HOBT hydrate (g) | NMP (mL) | HBTU (g) | NMP (mL) | DCM (mL0 | DIEA (mL) | Coupling time (min) |
| Lys(Boc) | 2.12 | 0.76 | 30.0 | 1.83 | 15.0 | 15.0 | 1.1 | 120 |
| Lys(Boc) recoupling | 2.12 | 0.76 | 30.0 | 1.83 | 15.0 | 15.0 | 1.1 | 120 |
| Val | 1.53 | 0.76 | 30.0 | 1.83 | 15.0 | 15.0 | 1.1 | 120 |
| Val recoupling | 1.53 | 0.76 | 30.0 | 1.83 | 15.0 | 15.0 | 1.1 | 120 |
| Leu | 1.58 | 0.76 | 30.0 | 1.83 | 15.0 | 15.0 | 1.1 | 120 |
| Leu | 1.58 | 0.76 | 30.0 | 1.83 | 15.0 | 15.0 | 1.1 | 120 |
| recoupling | | | | | | | | |
| Trp(Boc) | 2.37 | 0.76 | 30.0 | 1.83 | 15.0 | 15.0 | 1.1 | 120 |
| Trp(Boc) recoupling | 2.36 | 0.76 | 30.0 | 1.83 | 15.0 | 15.0 | 1.1 | 120 |
| Ala | 1.42 | 0.76 | 30.0 | 1.83 | 15.0 | 15.0 | 1.1 | 120 |
| Ala recoupling | 1.42 | 0.76 | 30.0 | 1.83 | 15.0 | 15.0 | 1.1 | 120 |
| Ile | 1.59 | 0.76 | 30.0 | 1.83 | 15.0 | 15.0 | 1.1 | 120 |
| Ile recoupling | 1.59 | 0.76 | 30.0 | 1.83 | 15.0 | 15.0 | 1.1 | 120 |
| Phe | 1.74 | 0.76 | 30.0 | 1.83 | 15.0 | 15.0 | 1.1 | 120 |
| Phe recoupling | 1.74 | 0.76 | 30.0 | 1.83 | 15.0 | 15.0 | 1.1 | 120 |
| Glu(OtBu) | 1.93 | 0.76 | 30.0 | 1.83 | 15.0 | 15.0 | 1.1 | 120 |
| Glu(OtBu) recoupling | 1.92 | 0.76 | 30.0 | 1.83 | 15.0 | 15.0 | 1.1 | 120 |
| Lys(Boc) | 2.12 | 0.76 | 30.0 | 1.83 | 15.0 | 15.0 | 1.1 | 120 |
| Lys(Boc) recoupling | 2.11 | 0.76 | 30.0 | 1.83 | 15.0 | 15.0 | 1.1 | 120 |
| Ala | 1.41 | 0.76 | 30.0 | 1.83 | 15.0 | 15.0 | 1.1 | 120 |
| Ala recoupling | 1.40 | 0.76 | 30.0 | 1.83 | 15.0 | 15.0 | 1.1 | 120 |
| Ala | 1.41 | 0.76 | 30.0 | 1.83 | 15.0 | 15.0 | 1.1 | 120 |
| Ala recoupling | 1.40 | 0.76 | 30.0 | 1.83 | 15.0 | 15.0 | 1.1 | 120 |
| Gln(trt) | 2.77 | 0.76 | 30.0 | 1.83 | 15.0 | 15.0 | 1.1 | 120 |
| Gln(trt) recoupling | 2.76 | 0.76 | 30.0 | 1.83 | 15.0 | 15.0 | 1.1 | 120 |

The built resin was isolated by washing with 4 times with NMP (6 vol.), 4 times with DCM (6 vol.), and 3 times with Isopropanol (IPA, 6 vol.). The built resin was dried at 35 °C under vacuum. 14.3 g built resin were obtained.

### C. Cleavage of the intermediate fragment from built resin

6.6 g of built resin from above were swelled in 10x volume DCM for 30min, and cooled to -10 °C. The DCM was drained and a cold solution of 1% TFA/DCM (12 vol. at -5 °C to -10 °C) was added and stirred for 30 min at 0 °C. The cleavage solution was collected in a flask containing pyridine (2-3 equiv. of TFA). While warming up to 25 °C, the resin was stirred with 1% TFA/DCM (10x vol.) for 5min and pyridine (2-3 equiv.) was added. After another 5 minutes, the solution was collected. The resin was washed with DCM 4 times (10 vol.). All DCM washes were combined with water (water/DCM = 1/4). The resultant mixture was distilled under reduced pressure to remove DCM (350 torr at 28 °C). The fragment precipitated out from water when DCM was removed. The fragment was washed with water and dried at 30 °C- 35°C under vacuum. The cleavage procedure was repeated one more time. A total of 2.36 g of Fmoc-(Aib³⁵) GLP-1 (23-35)-OH was obtained (a 92 % yield).

### Example 5

### A. Preparation of Fmoc-Aib-loaded 2CTC Resin

Fmoc-Aib-loaded 2CTC resin was prepared. The amounts of reagents used in this example are listed in following table:

| Preparation of Fmoc-Aib-2-Chlorotrityl Resin | | | | | |
|---|---|---|---|---|---|
| Materials | MW | Eq | mmol | grams | mL |
| 2-Chlorotritylchloride resin | - | - | 59.67 | 40.05 | - |
| Fmoc-Aib-OH | 325.5 | 1.0 | 14.92 | 4.85 | - |
| Diisopropylethylamine (DIEA) | 129.25 | 2.35 | 35.20 | 4.55 | |
| Dimethyl formamide (DMF) | | | | | 1280 |
| Dichloromethane (DCM) | | | | | 1840 |
| 9:1 Methanol: DIEA | | | | | 400 |
| Isopropanol (IPA) | | | | | 1050 |

2-CTC resin was charged to a 500 mL peptide reactor and swelled with 400 mL DCM for 30 min. The bed was drained, and a solution Fmoc-Aib-OH and DIEA in 8 volume of DMF:DCM (87.5:12.5) was added. The mixture was stirred under nitrogen for 2 hours at a temperature of 25 °C.

The bed was drained and washed with 400 mL DMF. Then, any remaining active sites on the 2-CTC resin were end-capped with 400 mL of MeOH:DIEA (9:1) solution for 1 hour. The bed was drained, washed one time with 400 mL DMF, one time with 200 mL DMF, and four times with 350 mL DCM. The resin was de-swelled by washing with 3x 350 mL IPA. The resin was dried to a constant weight to give 45.32 g of loaded resin. Analysis showed a loading factor of 0.30 mmol/g.

### B. Solid Phase Synthesis

Solid phase synthesis was carried out starting with 15.0g of Fmoc-Aib-2-CTC resin loaded at 0.30 mmol/g. The resin was swelled in DCM (150 mL) for 30 min at 25 °C. The DCM solvent was drained and the resin was washed two times with DCM (6 vol. each wash), and three times with NMP (6 vol. each wash).

The resin was then treated twice with 20% piperidine in NMP (6 vol. each treatment) to remove Fmoc protecting groups. After the second 20% piperidine/NMP treatment, the resin was washed six times with NMP (6 vol. each wash) to a negative chloranil test.

To prepare the coupling solution, the amino acid (1.7 equiv.) and 6-chloro-1-hydroxybenzotriazole (6-Cl-HOBT, 1.7 equiv.) were weighed, dissolved in 2.6x volume of NMP at 10 °C- 5 °C, and then combined with DIEA (1.9 to 3.0 equiv.). TBTU or HBTU( 1.7 equiv.) was dissolved in 1.33x volume of NMP at 10 °C- 5 °C. The two solutions were then combined. The resultant solution was added to a reaction vessel. The mixing flask was rinsed with 1.33x volume of DCM into the reactor, which was then stirred with resin for 2-3 hours at 25 °C- 27 °C. The sample was pulled for Kaiser Test to check the reaction completion. If the coupling reaction incomplete after 3 hours (positive Kaiser Test), the reaction vessel was drained, and recoupling was performed with fresh solution of activated amino acid. After the coupling reaction was completed, the coupling solution was drained and the resin was washed with NMP 4 times (6 vol. each wash). Then, the removal of the Fmoc group and coupling reaction cycle was repeated for the remaining amino acids in the fragment (i.e., in the order of Lys(Boc)→Val→Leu→ Trp(Boc)→Ala→Ile→Phe→Glu(OtBu)→Lys(Boc)→Ala→Ala→Gln(trt)).

Due to a possible buttressing effect between 2-methylalanine (Aib) and 2-CTC resin, there is considerable difficulty to force the first two amino acid coupling reactions (Lys(Boc)-34 and Val-33) to completion. Therefore, both coupling reactions for (Lys(Boc)-34, Val-33) were performed three times (i.e., coupling was followed by two recouplings). Also, acetic anhydride was used to end-cap the unreacted resin-bound material after coupling reactions of Lys(Boc)-34 and Val-33. This has improved the efficiency of the subsequent purification by moving the impurities far from the desirable product during chromatographic purification.

All reagents used in this example are listed in following table:

| Coupling Reaction of the Fmoc-AA(23-35)-OH | | | | | | | | | |
|---|---|---|---|---|---|---|---|---|---|
| Amino Acid | g/ Eq | 6-Cl-HOBT (g/Eq) | DIEA (g/Eq) | NMP (mL) | TBTU (g/Eq) | HBTU (g/Eq) | NMP (mL) | DCM (mL) | Coupl. time (min) |
| 1^{st} Lys(Boc) | 3.61 | 1.33 / | 1.15 / | 39.0 | 2.50 / | - | 20.0 | 20.0 | 175 |
| | / 1.7 | 1.7 | 1.9 | | 1.7 | | | | |
| 2^{nd} Lys(Boc) | 3.61 | 1.33 / | 1.16 / | 39.0 | 2.48 / | - | 20.0 | 20.0 | 180 |
| | / 1.7 | 1.7 | 1.9 | | 1.7 | | | | |
| 3^{rd} Lys(Boc) | 3.61 | 1.33 / | 1.13 / | 39.0 | 2.47 / | - | 20.0 | 20.0 | 180 |
| | / 1.7 | 1.7 | 1.9 | | 1.7 | | | | |
| Acetic Anhydride | 2.33 | - | 3.22 / | 60.0 | - | - | 30.0 | - | 120 |
| | / 5.0 | | 5.5 | | | | | | |
| 1^{st} Val | 2.62 | 1.33 / | 1.13 / | 39.0 | 2.51 / | - | 20.0 | 20.0 | 170 |
| | / 1.7 | 1.7 | 1.9 | | 1.7 | | | | |
| 2^{nd} Val | 2.62 | 1.33 / | 1.17 / | 39.0 | 2.49 / | - | 20.0 | 20.0 | 180 |
| | / 1.7 | 1.7 | 1.9 | | 1.7 | | | | |
| 3^{rd} Val | 2.63 | 1.32 / | 3.67 / | 39.0 | 2.50 / | - | 20.0 | 20.0 | 141 |
| | / 1.7 | 1.7 | 1.9 | | 1.7 | | | | |
| Acetic Anhydride | 4.69 | - | 7.13 / | 60.0 | - | - | 30.0 | - | 153 |
| | / 10.0 | | 12.0 | | | | | | |
| Leu | 2.73 | 1.35 / | 1.12 / | 39.0 | 2.50 / | - | 20.0 | 20.0 | 180 |
| | / 1.7 | 1.7 | 1.9 | | 1.7 | | | | |
| Trp(Boc) | 4.03 | 1.33 / | 1.78 / | 39.0 | 2.50 / | - | 20.0 | 20.0 | 180 |
| | / 1.7 | 1.7 | 3.0 | | 1.7 | | | | |
| Ala | 2.41 | 1.31 / | 1.78 / | 39.0 | - | 2.93 / | 20.0 | 20.0 | 180 |
| | / 1.7 | 1.7 | 3.0 | | | 1.7 | | | |
| Ile | 2.72 | 1.31 / | 1.78 / | 39.0 | - | 2.93 / | 20.0 | 20.0 | 180 |
| | / 1.7 | 1.7 | 3.0 | | | 1.7 | | | |
| Phe | 3.00 | 1.31 / | 1.78 / | 39.0 | - | 2.93 / | 20.0 | 20.0 | 180 |
| | / 1.7 | 1.7 | 3.0 | | | 1.7 | | | |
| Glu(OtBu) | 3.28 | 1.31 / | 1.78 / | 39.0 | - | 2.93 / | 20.0 | 20.0 | 180 |
| | / 1.7 | 1.7 | 3.0 | | | 1.7 | | | |
| Lys(Boc) | .3.61 | 1.31 / | 1.78 / | 39.0 | - | 2.93 / | 20.0 | 20.0 | 180 |
| | / 1.7 | 1.7 | 3.0 | | | 1.7 | | | |
| Ala | 2.40 | 1.31 / | 1.78 / | 39.0 | - | 2.93 / | 20.0 | 20.0 | 180 |
| | / 1.7 | 1.7 | 3.0 | | | 1.7 | | | |
| Ala | 2.41 | 1.31 / | 1.78 / | 39.0 | - | 2.93 / | 20.0 | 20.0 | 180 |
| | / 1.7 | 1.7 | 3.0 | | | 1.7 | | | |
| Gln(trt) | 4.72 | 1.31 / | 1.78 / | 39.0 | - | 2.93 / | 20.0 | 20.0 | 180 |
| | / 1.7 | 1.7 | 3.0 | | | 1.7 | | | |
| Gln(trt) | 4.72 | 1.31 / | 1.78 / | 39.0 | - | 2.93 / | 20.0 | 20.0 | 180 |
| | / 1.7 | 1.7 | 3.0 | | | 1.7 | | | |

### C. Cleavage of the fragment from the built resin

The built resin from above was washed with DCM 7 times (6 vol. each wash) to remove NMP residue, and the resin was cooled with the last DCM wash to -5 °C. The DCM was drained, and a cold solution of 1% TFA/DCM (12 vol. at -5 °C- to -10 °C) was added and stirred for 30 min at 0 °C. The cleavage solution was collected in a flask containing pyridine (1.3 equiv. of TFA). While the vessel warmed up to 25 °C, the resin was washed with DCM 9 times (10 vol.) and drained into the cleavage solution. The DCM solution was combined with water (6 vol.). The resultant mixture was distilled under reduced pressure to remove DCM (350 torr at 28 °C). The fragment precipitated out from water when DCM was removed. The fragment was washed with and dried at z °C to 35 °C under vacuum. For this example the cleavage procedure was repeated one more time. A total of 6.78 g of Fmoc-(Aib³⁵) GLP-1 (23-35)-OH was obtained (a 68.1% yield) with a purity of 87.3% AN..

### Example 6

### A. Preparation of Fmoc-Aib-loaded 2CTC Resin

Fmoc-Aib-loaded 2CTC resin was prepared. The amounts of reagents used in this example are listed in following table:

| Preparation of Fmoc-Aib-2-Chlorotrityl Resin | | | | | |
|---|---|---|---|---|---|
| Materials | MW | Eq | mmol | grams | mL |
| 2-Chlorotritylchloride resin | - | - | 59.85 | 40.44 | - |
| Fmoc-Aib-OH | 325.5 | 1.0 | 20.95 | 6.82 | - |
| Diisopropylethylamine (DIEA) | 129.25 | 0.95 | 19.88 | 2.57 | |
| Dimethyl formamide (DMF) | | | | | 1280 |
| Dichloromethane (DCM) | | | | | 1840 |
| 9:1 Methanol: DIEA | | | | | 400 |
| Isopropanol (IPA) | | | | | 1050 |

2-CTC resin was charged to a 500 mL peptide reactor and swelled with 400 DCM for 30 min. The bed was drained, and a solution of Fmoc-Aib-OH and DIEA in 8 volume of DMF:DCM (87.5:12.5) was added. The mixture was stirred under nitrogen for 2 hours at a temperature of 25 °C.

The bed was drained and washed with 400 mL DMF. Then, any remaining active sites on the 2-CTC resin were end-capped with 400 mL of MeOH:DIEA (9:1) solution for 1 hour. The bed was drained, washed one time with 400 mL DMF, washed one time with 200 mL DMF, and washed four times with 350 mL DCM. The resin was de-swelled by washing with 3x 350 mL IPA. The resin was dried to a constant weight to give 47.56 g of loaded resin. Analysis showed a loading factor of 0.37 mmol/g.

### B. Solid Phase Synthesis

Solid phase synthesis was carried out starting with 25.0g of Fmoc-Aib-2-CTC resin loaded at 0.37 mmol/g. The resin was swelled in DCM (250 mL) for 30 min at 25 °C. The DCM solvent was drained, and the resin was washed two times with DCM (6 vol. each wash), and three times with NMP (6 vol. each wash).

The resin was then treated twice with 20% by volume piperidine in NMP (6 vol. each treatment) to remove Fmoc protecting groups. After the second 20% piperidine/NMP treatment, the resin was washed six times with NMP (6 vol. each wash) to a negative chloranil test.

To prepare the coupling solution, the amino acid and 6-chloro-1-hydroxy-benzotriazole (6-Cl-HOBT) were weighed, dissolved in 3.2x volume of NMP (or DMF for Lys-34, Val-33, and Gln-23) then combined with DIEA at 10 °C to 5 °C. TBTU was dissolved in 1.6x volume of NMP (or DMF for Lys-34, Val-33, and Gln-23) at 10 °C to 5 °C. The two solutions were then combined. The resultant solution was added to reaction vessel, and the flask was rinsed with 1.6x volume of DCM into the reactor, which was stirred with resin for 2-3 hours at 25 °C to 27 °C. The sample was pulled for Kaiser Test to check the reaction completion. If the coupling reaction was incomplete after 3 hours (positive Kaiser Test), the reaction vessel was drained and recoupling was performed with fresh solution of activated amino acid. After the coupling reaction was completed, the coupling solution was drained, and the resin was washed with NMP 4 times (6 vol. each wash). Then, the deprotecting of the Fmoc group and coupling reaction cycle was repeated for remaining amino acid in the fragment (i.e., in the order of Lys(Boc)→Val→ Leu→Trp(Boc)→Ala→Ile→Phe→Glu(OtBu)→Lys(Boc)→Ala→Ala→Gln(trt)).

Due to a possible buttressing effect between 2-methylalanine (Aib) and 2-CTC resin, there is considerable difficulty to force the first two amino acid coupling reactions (Lys(Boc)-34 and Val-33) to completion. The coupling conditions for Lys(Boc)-34, Val-33, and Gln(trt)-23 were modified by increasing the usages of both amino acid and 6-Cl-HOBT from 1.7 Eq to 2.5 Eq and DIEA from 1.9 Eq to 3.0 Eq. The solvent for coupling reaction was also changed from NMP to DMF in order to force the coupling reaction to completion. Also, in this example, acetic anhydride was used to end-cap the unreacted resin-bound material after coupling reactions of Lys(Boc)-34 and Val-33. This has improved the efficiency of the subsequent purification by moving the impurities far from the desirable product during chromatographic purification.

All reagents used in this example are listed in following table:

| Coupling Reaction of the Fmoc-AA(23-35)-OH | | | | | | | | | | |
|---|---|---|---|---|---|---|---|---|---|---|
| Material | wt (g) / Eq | 6-Cl-HOBT (g/Eq) | DIEA (g/Eq) | DMF (mL) | NMP (mL) | TBTU (g/Eq) | DMF (mL) | NMP (mL) | DCM (mL) | Coupl. time (min) |
| Lys(Boc) | 10.8 | 3.93 / | 3.63 / | 80.0 | - | 7.44 / | 40.0 | - | 40.0 | 170 |
| | 4 / 2.5 | 2.5 | 3.0 | | | 2.5 | | | | |
| Acetic Anhydride | 4.72 | - | 6.61 / | - | 100.0 | - | - | 50.0 | - | 120 |
| | /5.0 | | 5.5 | | | | | | | |
| Val | 7.85 | 3.92 / | 3.67 / | 80.0 | - | 7.44 / | 40.0 | - | 40.0 | 177 |
| | /2.5 | 2.5 | 3.0 | | | 2.5 | | | | |
| Acetic Anhydride | 9.48 / 10.0 | | 14.46 /12.0 | - | 100.0 | - | - | 50.0 | - | 120 |
| Leu | 5.56 | 2.68 / | 2.33 / | - | 78.6 | 5.05 / | - | 39.3 | 39.3 | 184 |
| | /1.7 | 1.7 | 1.9 | | | 1.7 | | | | |
| Trp(Boc) | 8.30 | 2.70 / | 2.28 / | - | 78.6 | 5.05 / | - | 39.3 | 39.3 | 180 |
| | /1.7 | 1.7 | 1.9 | | | 1.7 | | | | |
| Ala | 4.92 | 2.68 / | 2.30 / | - | 78.6 | 5.05 / | - | 39.3 | 39.3 | 177 |
| | /1.7 | 1.7 | 1.9 | | | 1.7 | | | | |
| Ile | 5.56 | 2.70 / | 2.26 / | - | 78.6 | 5.06 / | - | 39.3 | 39.3 | 168 |
| | /1.7 | 1.7 | 1.9 | | | 1.7 | | | | |
| Phe | 6.10 | 2.70 / | 2.31 / | - | 78.6 | 5.06 / | - | 39.3 | 39.3 | 168 |
| | /1.7 | 1.7 | 1.9 | | | 1.7 | | | | |
| Glu(OtBu) | 6.72 | 2.67 / | 2.29 / | - | 78.6 | 5.05 / | - | 39.3 | 39.3 | 168 |
| | /1.7 | 1.7 | 1.9 | | | 1.7 | | | | |
| Lys(Boc) | 7.39 | 2.70 / | 2.29 / | - | 78.6 | 5.05 / | - | 39.3 | 39.3 | 165 |
| | /1.7 | 1.7 | 1.9 | | | 1.7 | | | | |
| Ala | 4.91 | 2.70 / | 2.41 / | - | 78.6 | 5.05 / | - | 39.3 | 39.3 | 180 |
| | /1.7 | 1.7 | 1.9 | | | 1.7 | | | | |
| Ala | 4.92 | 2.68 / | 2.32 / | - | 78.6 | 5.03 / | - | 39.3 | 39.3 | 171 |
| | /1.7 | 1.7 | 1.9 | | | 1.7 | | | | |
| Gln(trt) | 14.1 | 3.94 / | 3.71 / | 80.0 | - | 7.42 / | 40.0 | - | 40.0 | 185 |
| | 3 / | 2.5 | 3.0 | | | 2.5 | | | | |
| | 2.5 | | | | | | | | | |

### C. Cleavage of the fragment from built resin

The built resin from above was washed with DCM 6 times (6 vol. each wash) to remove NMP, and the resin was cooled with the last DCM wash to -5 °C. The DCM was drained, and a cold solution of 1% TFA/DCM (10 vol. at -5 °C to -10 °C) was added and stirred for 30 min at 0 °C. The cleavage solution was collected in a flask containing pyridine (1.3 equiv. of TFA). While the vessel warmed up to 25 °C, the resin was washed with DCM 7 times (6 vol.) and drained into the cleavage solution. The DCM solution was combined with water (10 vol.). The resultant mixture was distilled under reduced pressure to remove DCM (350 torr at 28 °C). The fragment precipitated out from water when DCM was removed. The fragment was washed with and dried at 30 °C to - 35 °C under vacuum. For this example, the cleavage procedure was repeated one more time to achieve complete cleavage. A total of 12.36 g of Fmoc-(Aib³⁵) GLP-1 (23-35)-OH was obtained (a 59.35% yield) with a purity of 84.3% AN.

### Example 7

### 1. Resin Washing

Starting with preloaded Fmoc-Gly-O-2-CTC resin (loading from 0.18 to 0.65 mmol/g), or Fmoc-Aib-O-2-CTC (loading 0.25 to 0.65 mmol/g), standard Fmoc chemistry applied. The resin was first swelled in 10x volume of DCM for 30-60 min. Then DCM was drained and the resin was washed with 10x volume of NMP for 3-5 times (5 min each).

### 2. General Synthesis Cycle:

Using a resin washed in accordance with Section A of this Example 7, Fmoc removal was accomplished by two treatments of ∼10x solution of 20% Piperidine in NMP (v/v). The treatments lasted 15-30 min/each. The Piperidine/NMP solution was drained after each treatment. The resin was then washed by NMP 4-5 times (10x volume, 5 min/each).

To prepare the coupling solution, the Fmoc protected amino acid (AA), and HOBt, were weighed (at 1.5-2.0 equiv), dissolved in 4x volume of NMP at 10°C, and combined with DIEA (1.5-2.0 equiv). HBTU (1.5-2.0 equiv) was dissolved in 3x volume of NMP at 10°C. The two solutions were then combined and mixed with 3x volume of DCM for 1-2 min. The resultant solution was added to the reaction vessel and mixed with the resin under agitation for 1.5-5 hours. The sample was pulled for Kaiser' test to check the reaction for completion. Uncompleted coupling was recoupled. After the coupling reaction was completed, the coupling solution was drained and the resin was washed with NMP 5 times (10x volume, 5 min/each).
A. Following the general synthesis procedure, the fragment Fmoc- GLP-1 (11-22)-2-CTC having the native sequence was built stepwise using the same general synthesis procedure, a fragment according to SEQ ID NO. 7 was coupled to the resultant fragment Fmoc-GLP-1 (11-22)-2-CTC prepared in this Section A using the general synthesis procedure.
B. Following the general synthesis procedure, the fragment Fmoc-GLP-1 (11-22)-2-CTC was built stepwise. However, the Fmoc removal condition was changed to a solution of 10% piperidine and 2% DBU in NMP(v/v) instead of 20% piperidine in NMP (v/v). In addition, the treatment times at the position of AA14 [Fmoc-Ser(tBu)-] and the position of AA13 [Fmoc-Thr(tBu)-] were extended to 1.5 h to drive the reaction completion. Using the same general synthesis procedure, a fragment according to SEQ ID NO. 7 was coupled to the resultant Fmoc-GLP-1 (11-22)-2-CTC using the general synthesis procedure.
C. Following the general synthesis procedure, with the Fmoc removal solution as 10% piperidine and 2% DBU in NMP(v/v), the Fragment Fmoc-(X¹⁷⁻¹⁸) GLP-1 (11-22)-2-CTC was built stepwise. Then the fragment according to SEQ ID NO. 7 was coupled to the resultant Fmoc-(X¹⁷⁻¹⁸) GLP-1 (11-22)-2-CTC using the general synthesis procedure.
D. Following the general synthesis procedure, the fragment Fmoc-(X¹⁷⁻¹⁸) GLP-1 (11-22)-2-CTC was built stepwise. Then the fragment according to SEQ ID NO. 7 was coupled to the resultant Fmoc-(X¹⁷⁻¹⁸) GLP-1 (11-22)-2-CTC using the general synthesis procedure.
E. Following the general synthesis procedure of section D immediately above, and except for differences noted in the following table, a fragment Fmoc-(X¹⁷⁻¹⁸) GLP-1 (11-22)-2-CTC was built stepwise. Then a fragment according to SEQ ID NO. 7 was coupled to the resultant Fmoc-(X¹⁷⁻¹⁸) GLP-1 (11-22)-2-CTC fragment using the general synthesis procedure.

The following table summarizes details of procedures A through E of this Example:

| **Ex.** | **Scale** | **Loading mmol/g** | **Fmoc Removal Condition** | **Pseudoproline @ Position 17-18** | **Equiv. of AA** | **Purity HPLC** | **Yield** |
|---|---|---|---|---|---|---|---|
| **A** | 5g | 0.24 | 20% piperidine/ | No | 2.0 | 30% | 42%* |
| | | | NMP | | | | |
| **B** | 20g | 0.30 | 10% piperidine/ | No | 1.7 | 58% | 62% |
| | | | 2%DBU/NMP | | | | |
| **C** | 5g | 0.30 | 10% piperidine/ | Yes | 2.0 | 84% | 74% |
| | | | 2%DBU/NMP | | | | |
| **D** | 5g | 0.30 | 20% piperidine/ | Yes | 2.0 | 75% | 80% |
| | | | NMP | | | | |
| **E** | 20g | 0.42 | 20% piperidine/ | Yes | 2.0 | 89% | 86% |
| | | | NMP | | | | |

### F. The Fragment Cleavage:

To accomplish fragment cleavage with respect to any of the peptide fragments synthesized in this Example, the built peptide-resin is swelled in 10x volume of DCM for 30 min, and is cooled to -10 °C. The DCM is drained and a solution of 1% TFA/DCM (10x volume) is added and stirred for 30 min. The cleavage solution is collected in a flask containing pyridine (2-3 equiv. relative to TFA). While warming up to 25 °C, the resin is treated with 1% TFA/DCM (10x volume) for 5 min, and then pyridine (2-3 equiv. to TFA) is added. After another 5 min agitation, the solution is collected. The resin is then washed with 10x volume of DCM for 4 times (5 min/each). The solutions of all the washes and the cleavage are combined and mixed with water (water/DCM ratio = ∼1/4 by volume). The resultant mixture is distilled at reduced pressure to remove DCM (350 torr/28 °C). The peptide fragment crashes out from water when DCM is removed, and is filtered. The peptide fragment is washed with water and dried at 30 °C under vacuum.

### Example 8 - Solution Synthesis: Adding Arg

A (Aib³⁵) GLP-1 (23-35) fragment (bearing side chain protection according to Table A and bearing Fmoc protection at the N-terminus (9.11 g, 3.94 mmol) was dissolved in DMSO (90 mL). To this solution, HOBt (2.42 g, 4 equiv), HBTU (5.98 g, 4 equiv), DIEA (3.44 mL, 5 equiv), and H-Arg (2HCl)-NH2 (3.8 8g, 4 equiv) were charged along with 10 mL of DMSO. The reaction was agitated and monitored by HPLC. After 4 hours, the reaction was not completed, so 2 mL of DIEA was added. The reaction was done overnight. Then piperidine (5 mL) was added to the reaction mixture. The Fmoc removal was done in 2 hours. The reaction mixture was quenched with ice water (800 mL) and stirred for 40 min. The white solid formed was filtered, washed with water (400 mL) and dried overnight to give the fragment (9.65 g, weight yield 109%) (Aib³⁵) GLP-1 (23-36).

### Example 9

The fragment (Aib⁸, X¹⁷⁻¹⁸) GLP-1 (7-22) (7.73 g, 2.88 mmol) bearing side chain protection groups in accordance with Table A and Fmoc protection at the N-termines was dissolved in DMSO (65 mL).

To this solution, HOBt (0.73 g, 4.77 mmol), HBTU (1.46 g, 3.85 mmol), DIEA (0.71 mL, 7.40 mmol), and the Fragment (Aib³⁵) GLP-1 (23-36) (8.5 g, 3.45 mmol) were charged along with 20 mL of DMSO. The reaction was agitated and monitored by HPLC. After 3 hours the coupling was completed. Then piperidine (5 mL) was added to the reaction mixture. The Fmoc removal was done in 2 hours. The reaction mixture was quenched with ice water (800 mL) and stirred for 30 min. The white solid formed was filtered, washed with water (400 mL) and dried overnight to give the protected peptide (Aib⁸, X¹⁷⁻¹⁸, Aib³⁵) GLP-1 (7-36).

### Example 10 - Global Deprotection

A peptide prepared according to Example 9 (16.24 g) was treated with a solution of TFA/DTT/Water (100 mL/5 g/2.0 mL) for 2 hours and the resultant solution was poured into MTBE (800 mL) in ice bath. After 30 min agitation, the white solid formed was filtered, washed with MTBE (400 mL) and dried to provide the crude peptide product (16.0 g, weight yield 138%). The resultant de-protected peptide is hereinafter referred to as the "crude" peptide.

### Example 11 - Purification

Purification of the crude peptide is performed on a Kromasil^{®} C4, 10 micron, 2.0 x 25 cm column yielding purified peptide at 98+% purity and ∼60 % contained / contained yield. The purification involves a 1st pass chromatographic purification at pH 2, followed by a 2nd pass at pH 9. After this purification, the purified peptide may be further handled or processed in a variety of ways. By way of example, the resulting purified pool from the second pass may be lyophilized or passed through a concentration column and isolated by precipitation. Both isolations will yield the desired peptide (acetate).

As an overview of the two pass process, crude peptide is dissolved at 5 mg/ml (contained basis) in a mixture of 10% acetonitrile / water (0.2M Acetic Acid). Replicate 1000 mg injections (contained basis) are made using an acetonitrile / THF / water /TFA gradient, and fractions of ∼95% purity are pooled. A "recycle fraction" is also pooled at ∼70% purity representing ∼34% recovery. The recycle fraction is re-injected using the same acetonitrile / THF / water / TFA gradient, and a pool at ∼85% purity is combined with the main pool. The contained to contained yield for the 1st pass chromatographic purification is 70%.

The combined 1st pass pool (pH 2) was then further purified in a second pass on the same Kromasil^{®} C4 column but using an acetonitrile / THF / water / ammonium acetate (pH 8.8) gradient. Fractions are combined yielding 98 +% purity at a ∼85% 2nd pass recovery. The overall yield for both purification steps will be 60% (contained /contained).

The combined 2nd pass pool (pH 8.8) was then concentrated using the same Kromasil^{®} C4 column but using a methanol / water / ammonium acetate step gradient. Fractions are combined and are ready for isolation.

### A. Crude peptide solution preparation:

8 g of crude peptide was dissolved in 500 ml of a solution of 90% 0.2N acetic acid /10% acetonitrile. The solution was filtered, once through a 0.45 micron Durapore filter (47 mm diameter) (Millipore) and then through a Supor EKV disk stack micron filter (0.65 / 0.2 mm diameter) (Pall Filters). The crude injection solution was analyzed by HPLC and found to contain 5.02 mg (wt%) of contained peptide per ml. (500 ml x 5.02 mg/ml = 2512 mg contained peptide).

### B. Chromatography - 1st pass at pH ~2:

Four replicate injections were made with the crude solution under the following conditions:
Column: Manufactured by Kromasil with a packing of C4, 10 micron and the dimensions of 2.0 x 25 cm
Detector: Ultraviolet detector set to 280 nm (8 nm bandwidth, 350/20 nm ref)
Column Temp: ambient
Flow rate: 13.0 ml/min (~50 bar back pressure)
Mobile phase:
   A = mixture of 0.1% trifluoroacetic acid /15% acetonitrile / 85% water
   B = mixture of 0.1% trifluoroacetic acid / 15% tetrahydrofuran / 70% acetonitrile /15% water
Gradient: The gradient begins with 100% A mobile phase and is held for 0.1 minute. The sample is then manually loaded onto the column by pump C (see below for description of Pump C). After sample loading, a linear gradient from 100% A to 83% A is run over 1 minute. The mobile phase is then held at 83% A for the next 11 minutes. A second linear gradient is then run to 73% A over 10 minutes. The mobile phase is then held at 73% A for the next 15 minutes. The run is then complete and is followed by a 10 minute 100% B flush followed by a 20 minute re-equilibration of the column at 100% A.
   The sample was loaded using a separate isocratic HP 1100 pump at 9.0 ml/min (pump - C). The early eluting impurities are separated from the main peak by an initial 11 minute isocratic hold at 83% A followed by a linear gradient to 73% A over the next 10 minutes. The main peptide peak is then eluted during the 15 minute hold at 73% A. Fractions are collected during this 15 minute hold at 73% A.

### C. 1^{st} Pass at pH ~2 Recycle:

The recycle pool is obtained from fractions determined to have purities below that which can be added to the combined pool. These fractions were combined separately and diluted with an equal volume of water. A recycle injection was made back onto the column from this separately combined pool. The same chromatographic conditions are used and the fractions of acceptable purity are combined, diluted with and equal volume of water, and added to the 1st pass combined pool.

Loading for the recycle injection is significantly less than with the crude injection due to the increased impurities which elute near the peptide peak. On average there is 1 recycle injection for every 2 crude injections.

### D. 2^{nd} Pass Prep Chromatography at pH 8.8:

The final combined 1st pass pool was further purified by re-chromatographing at pH 8.8. Using pH 8.8 for the 2nd pass significantly changed the elution order of the impurities, enabling a better clean-up at higher recovery. The conditions used for this 2nd chromatography step were as follows:
Column: Manufactured by Kromasil with a packing of C4, 10 micron and the dimensions of 2.0 x 25cm
Detector: Ultraviolet detector set to 280 nm (8 nm bandwidth, 350/20 nm ref)
Column Temp: ambient
Flow rate: 13.0 ml/min (∼50 bar back pressure)
Mobile Phase:
   A = mixture of 15% acetonitrile / 85% water containing 2 grams per liter of ammonium acetate and 1mL per liter of concentrated ammonium hydroxide
   B = mixture of 15% tetrahydrofuran / 60% acetonitrile / 25% water containing 2 grams per liter of ammonium acetate and 1mL per liter of concentrated ammonium hydroxide.
Gradient: The gradient begins with 100% A mobile phase and is held for 0.1 minute. The sample is then manually loaded onto the column by pump C. After sample loading, a linear gradient from 100% A to 67% A is run over 2 minutes. The mobile phase is then held at 67% A for the next 33 minutes. The run is then complete and is followed by a 5 minute 100% B flush followed by a 15 minute re-equilibration of the column at 100% A.
   The sample was loaded using a separate isocratic HP 1100 pump at 9.0 ml/min (pump - C). After the 30 minute loading step, a short gradient from 100%A to 67%
   A was run from 30.2 to 32 min. The main peptide peak was eluted during a 33 minute hold at 67% A. Fractions are collected starting 20 minutes after column loading and end after the main peak has completed eluting. The fractions are collected for approximately 15 minutes. Acceptable fractions are pooled and diluted with an equal volume of water. A recycle is possible at this stage of the purification but the waste fractions generally do not contain enough peptide to warrant a recycle injection. This may become feasible if a large number of injections are made and the waste fractions pooled for recycle.

### E. Concentration Run:

The final combined 2nd pass combined pool is loaded onto the same column and eluted quickly using a different mobile phase to concentrate peptide for isolation. The conditions used for this step were as follows:
Column: Manufactured by Kromasil with a packing of C4, 10 micron and the dimensions of 2.0x25 cm
Detector: Ultraviolet detector set to 280 nm (8 nm bandwidth, 350/20 nm ref)
Column Temp: ambient
Flow rate: 13.0 ml/min (∼50 bar back pressure)
Mobile phase:
   A = mixture of 10% methanol / 90% 20 mM ammonium acetate
   B = mixture of 90% methanol / 10% 20 mM ammonium acetate
Gradient: The gradient begins with 100% A mobile phase and is held for 0.1 minute. The sample is then manually loaded onto the column by pump C. After sample loading, the mobile phase is held at 100% A for 5 minutes. The mobile phase is then immediately stepped to 100%B for the next 20 minutes. The run is then complete and is followed by a 15 minute re-equilibration of the column at 100% A.
   The sample was loaded using a separate isocratic HP 1100 pump at 9.0 ml/min (pump - C). After the 45 minute loading step, a short hold using 100% A for 5 minutes then a step gradient to 100% B was run for 20 minutes to elute the peptide.
   The main peak began eluting 2 minutes after the step gradient to 100%B. The next 12 minutes of fractions contained peptide and were pooled for isolation.

### Example 12 - Lyophilization

A 1 liter wide mouthed FLPE bottle was tared. To this bottle was added the purified pool (210 ml) of (Aib⁸, X¹⁷⁻¹⁸, Aib³⁵) GLP-1 (7-36) acetate in aqueous acetonitrile/ammonium acetate. The container was rinsed with deionized water 3 x 5 mL and the rinses were added to the pool. This solution was swirled to homogenize and then capped and shell frozen in liquid nitrogen. The frozen bottle was uncapped and the mouth covered with a doubled Kimwipe held in place with a rubber band. The bottle was placed in a vacutainer on the lyophilizer and lyophilized. Condenser temperature -89°C, pressure 19 microns.

After 24 h the vacutainer was vented to check the progress; there was still an audible chunk of ice present. Lyophilization restarted.

After a further 18 h the bottle was removed from the lyophilizer and the weight was checked. The weight was unstable, rising rapidly due to the hygroscopic nature of the product. The staticky product was quickly transferred to a tared scintillation vial and weighed, 0.822 g of peptide was obtained.

### Example 13: Precipitation of purified peptide

In a flask, a solution of 100.5 mg of pure (Aib⁸, X¹⁷⁻¹⁸, Aib³⁵) GLP-1 (7-36) acetate in 2 mL 20 mM ammonium acetate in MeOH/water (9:1 by volume) was diluted with 1 mL 20mM ammonium acetate in MeOH/water (9:1 by volume) With agitation, 20 mL Isopropanol (IPA) were slowly fed into flask at 20 °C to 25°C. The pot mixture became cloudy after adding 15 mL IPA. Stirring continued overnight at 20 °C to 25 °C. The precipitating product was filtered and washed by 5 mL IPA and then dried at 25°C under vacuum until a constant weight. 90.9 mg peptide was obtained (a 90.42% recovery).

### Example 14: Isolation of purified peptide

In a flask, a solution of 497.7 mg of pure (Aib⁸, ^{X17-18}, Aib³⁵) GLP-1 (7-36) acetate in 10 mL 20 mM ammonium acetate in MeOH/water (9:1) was diluted with 6 mL 20mM ammonium acetate in MeOH/water (9:1). With agitation, 40 mL isopropanol (IPA) were slowly fed into the flask at 20 °C to 25 °C over 35 min. The pot mixture became cloudy after adding 2 mL IPA. Stirring continued for an hour at 20 °C to 25°C. The precipitating product was filtered and washed by 5 mL IPA and then dried at 25 °C under vacuum until a constant weight. 458.4 mg peptide was obtained (a 92% recovery).

### Example 15: Isolation of purified peptide

In a flask, 900 mL IPA were slowly added into a stirring solution of ~ 1000 mg of purified (Aib⁸, ^{X17-18}, Aib³⁵) GLP-1 (7-36) acetate in 150 mL 20 mM ammonium acetate in MeOH/water (9:1) through a concentration column at 20 °C to 25°C. This addition was complete over 45 min. The pot mixture became cloudy after adding 260 mL IPA. Stirring continued for 40 min at 20 °C to 25°C. The precipitating product was filtered and washed by 5 mL IPA and then dried at 20 °C to 25 °C under vacuum until a constant weight. A 746 mg peptide was obtained (a 74.6% recovery).

## Claims

1. A method of making an insulinotropic peptide, comprising the following steps of
a) to f):
a) providing a first peptide fragment including the amino acid sequence HX⁸EX¹⁰ (SEQ ID NO. 6), wherein X⁸ and X¹⁰ are each residues of an achiral amino acid, each of H and E optionally including side chain protection;
b) providing a second peptide fragment including the amino acid sequence TFTSDVX¹⁷⁻¹⁸YLEG (SEQ ID NO. 8) wherein the residue denoted by the symbol X¹⁷⁻¹⁸ is a dipeptide residue of a pseudoproline, said amino acid residues of the sequence optionally including side chain protection;
c) coupling the first fragment to the second fragment to provide a third peptide fragment including the amino acid sequence HX⁸EX¹⁰ TFTSDVX¹⁷⁻¹⁸YLEG (SEQ ID NO. 11), said amino acid residues of the sequence optionally including side chain protection;
d) providing a fourth peptide fragment including the amino acid sequence QAAKEFIAWLVKX³⁵ (SEQ ID NO. 9), wherein X³⁵ is a residue of an achiral amino acid, said amino acid residues of the sequence optionally including side chain protection;
e) coupling the fourth peptide fragment to arginine in order to provide a fifth peptide fragment including the amino acid sequence QAAKEFIAWLVK X³⁵R (SEQ ID NO. 12), said residues of the sequence optionally including side chain protection; and
f) coupling the fifth fragment to the third fragment in order to provide an insulinotropic peptide including the amino acid sequence HX⁸EX¹⁰TFTSDVX¹⁷⁻¹⁸YLEGQAAKEFIAWLVK X³⁵R (SEQ ID NO. 13), said residues of the sequence optionally including side chain protection.

2. The method according to claim 1, comprising a further step of:
g) removing the side chain protecting groups in order to provide an insulinotropic peptide including the amino acid sequence HX⁸EX¹⁰TFTSDVSSYLEGQAAKEFIAWLVKX³⁵R (SEQ ID NO. 5) and counterparts thereof, wherein each of the symbols X at positions, 8, 10, and 35 independently denotes an achiral, optionally sterically hindered amino acid residue.

3. A method of making an insulinotropic peptide according to claim 1, comprising the steps of:
a) providing a first peptide fragment including the amino acid sequence HX⁸EX¹⁰ (SEQ ID NO. 6), wherein X⁸ is an amino acid residue corresponding to Aib and X¹⁰ is an amino acid residue corresponding to glycine, and each of H and E optionally including side chain protection;
b) providing a second peptide fragment including the amino acid sequence TFTSDVX¹⁷⁻¹⁸YLEG (SEQ ID NO. 8) wherein the residue denoted by the symbol X¹⁷⁻¹⁸ is a dipeptide residue of a pseudoproline, said amino acid residues of the sequence optionally including side chain protection;
c) coupling the first fragment to the second fragment to provide a third peptide fragment including the amino acid sequence HX⁸EX¹⁰ TFTSDV X¹⁷⁻¹⁸YLEG (SEQ ID NO. 11), said amino acid residues of the sequence optionally including side chain protection;
d) providing a fourth peptide fragment including the amino acid sequence QAAKEFIAWLVKX³⁵ (SEQ ID NO. 9), wherein X³⁵ is an amino acid residue corresponding to Aib, said amino acid residues of the sequence optionally including side chain protection;
e) coupling the fourth peptide fragment to arginine in order to provide a fifth peptide fragment including the amino acid sequence QAAKEFIAWLVK X³⁵R (SEQ ID NO. 12), said residues of the sequence optionally including side chain protection; and
f) coupling the fifth fragment to the third fragment followed by removing the side chain protecting groups in order to provide an insulinotropic peptide of the formula HX⁸EX¹⁰TFTSDVSSYLEGQAAKEFIAWLVKX³⁵R (SEQ. ID No. 5) and counterparts thereof, wherein X⁸ and X³⁵ are amino acid residues corresponding to Aib and X¹⁰ is an amino acid residue corresponding to glycine.

4. The method according to claims 1 or 3, wherein X⁸ is an amino acid residue corresponding to methyl alanine.

5. The method according to claims 1 or 3, wherein X¹⁰ is an amino acid residue corresponding to glycine.

6. A peptide fragment having the amino acid sequence HX⁸EX¹⁰ (SEQ ID NO. 6), wherein X⁸ and X¹⁰ are each residues of an achiral amino acid, each of H, E, X⁸ and X¹⁰ optionally including side chain protection.

7. The peptide fragment of claim 6, wherein X⁸ is an amino acid residue corresponding to Aib and X¹⁰ is an amino acid residue corresponding to glycine.

8. The method according to claim 1, wherein the insulinotropic peptide includes the amino acid sequence (SEQ. ID No. 5)
HX⁸EX¹⁰TFTSDVSSYLEGQAAKEFIAWLVKX³⁵R
and counterparts thereof, wherein each of the symbols X at positions, 8, 10, and 35 independently denotes an achiral, optionally sterically hindered amino acid residue; and wherein one or more of the amino acid residues optionally includes side chain protection.

9. The method according to claim 8, wherein at least one of X⁸ and X³⁵ is a residue of Aib.

10. The method according to claim 8, wherein X¹⁰ is a residue of glycine.

11. The method according to claim 1, wherein X¹⁷⁻¹⁸ has the formula 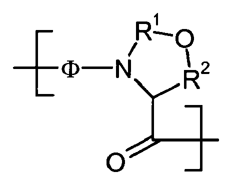 wherein Φ represents the residue of any amino acid optionally include side chain protection and each of R¹ and R² is independently a suitable divalent linking moiety.

12. The method according to claim 11, wherein Φ represents a residue of Ser optionally including side chain protection.

13. The method according to claim 11, wherein R² is -CH₂-.

14. The method according to claim 11, wherein R¹ is 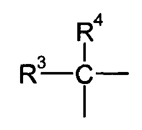 wherein each of R³ and R⁴ is independently a monovalent moiety selected from H or lower alkyl; or R³ and R⁴ also may be co-members of a ring structure.

15. The method according to claim 14, wherein each of R³ and R⁴ is methyl.

16. A peptide or a counterpart thereof including the amino acid sequence TFTSDVX¹⁷⁻¹⁸YLEG (SEQ. ID NO. 8) wherein the residue denoted by the symbol X¹⁷⁻¹⁸ is a dipeptide residue of a pseudoproline; said amino acid residues optionally including side chain protection.

17. The method according to claim 1, wherein X³⁵ is an amino acid residue of methylalanine.

18. The method according to claims 1 to 2, wherein the insulinotropic peptide has the amino acid sequence (SEQ. ID No. 4)
HAibEGTFTSDVSSYLEGQAAKEFIAWLVKAibR
and counterparts thereof.

19. The method according to claim 18, wherein the insulinotropic peptide has the amino acid sequence (SEQ. ID No. 4)
HAibEGTFTSDVSSYLEGQAAKEFIAWLVKAibR
and counterparts thereof, which is amidated at the C-terminus.

## Patentansprüche

1. Verfahren zur Herstellung eines insulinotropen Peptids, umfassend die folgenden Schritte
a) bis f):
a) Bereitstellen eines ersten Peptidfragments, das die Aminosäuresequenz HX⁸EX¹⁰ (SEQ ID NR.: 6) umfasst, wobei X⁸ und X¹⁰ jeweils Reste einer achiralen Aminosäure sind, wobei jeder von H und E gegebenenfalls Seitenkettenschützung umfasst;
b) Bereitstellen eines zweiten Peptidfragments, das die Aminosäuresequenz TFTSDVX¹⁷⁻¹⁸YLEG (SEQ ID NR.: 8) umfasst, wobei der Rest, der mit dem Symbol X¹⁷⁻¹⁸ bezeichnet wird, ein Dipeptidrest eines Pseudoprolins ist, wobei die Aminosäurereste der Sequenz gegebenenfalls Seitenkettenschützung umfassen;
c) Koppeln des ersten Fragments an das zweite Fragment zur Bereitstellung eines dritten Peptidfragments, das die Aminosäuresequenz HX⁸EX¹⁰TFTSDVX¹⁷⁻¹⁸YLEG (SEQ ID NR.: 11) umfasst, wobei die Aminosäurereste der Sequenz gegebenenfalls Seitenkettenschützung umfassen;
d) Bereitstellen eines vierten Peptidfragments, das die Aminosäuresequenz QAAKEFIAWLVKX³⁵ (SEQ ID NR.: 9) umfasst, wobei X³⁵ ein Rest einer achiralen Aminosäure ist, wobei die Aminosäurereste der Sequenz gegebenenfalls Seitenkettenschützung umfassen;
e) Koppeln des vierten Peptidfragments an Arginin zur Bereitstellung eines fünften Peptidfragments, das die Aminosäuresequenz QAAKEFIAWLVKX³⁵R (SEQ ID NR.: 12) umfasst, wobei die Reste der Sequenz gegebenenfalls Seitenkettenschützung umfassen; und
f) Koppeln des fünften Fragments an das dritte Fragment zur Bereitstellung eines insulinotropen Peptids, das die Aminosäuresequenz HX⁸EX¹⁰TFTSDVX¹⁷⁻¹⁸YLEGQAAKEFIAWLVKX³⁵R (SEQ ID NR.: 13) umfasst, wobei die Reste der Sequenz gegebenenfalls Seitenkettenschützung umfassen.

2. Verfahren nach Anspruch 1, umfassend einen weiteren Schritt des:
g) Entfernens der Seitenkettenschutzgruppen zur Bereitstellung eines insulinotropen Peptids, das die Aminosäuresequenz HX⁸EX¹⁰TFTSDVSSYLEGQAAKEFIAWLVKX³⁵R (SEQ ID NR.: 5) und Gegenstücke davon umfasst, wobei jedes der Symbole X an den Positionen 8, 10 und 35 unabhängig einen achiralen, gegebenenfalls sterisch gehinderten Aminosäurerest bezeichnet.

3. Verfahren zur Herstellung eines insulinotropen Peptids nach Anspruch 1, umfassend die Schritte:
a) Bereitstellen eines ersten Peptidfragments, das die Aminosäuresequenz HX⁸EX¹⁰ (SEQ ID NR.: 6) umfasst, wobei X⁸ ein Aminosäurerest ist, der Aib entspricht, und X¹⁰ ein Aminosäurerest ist, der Glycin entspricht, und jeder von H und E gegebenenfalls Seitenkettenschützung umfassen;
b) Bereitstellen eines zweiten Peptidfragments, das die Aminosäuresequenz TFTSDVX¹⁷⁻¹⁸YLEG (SEQ ID NR.: 8) umfasst, wobei der Rest, der mit dem Symbol X¹⁷⁻¹⁸ bezeichnet wird, ein Dipeptidrest eines Pseudoprolins ist, wobei die Aminosäurereste der Sequenz gegebenenfalls Seitenkettenschützung umfassen;
c) Koppeln des ersten Fragments an das zweite Fragment zur Bereitstellung eines dritten Peptidfragments, das die Aminosäuresequenz HX⁸EX¹⁰TFTSDVX¹⁷⁻¹⁸YLEG (SEQ ID NR.: 11) umfasst, wobei die Aminosäurereste der Sequenz gegebenenfalls Seitenkettenschützung umfassen;
d) Bereitstellen eines vierten Peptidfragments, das die Aminosäuresequenz QAAKEFIAWLVKX³⁵ (SEQ ID NR.: 9) umfasst, wobei X³⁵ ein Aminosäurerest ist, der Aib entspricht, wobei die Aminosäurereste der Sequenz gegebenenfalls Seitenkettenschützung umfassen;
e) Koppeln des vierten Peptidfragments an Arginin zur Bereitstellung eines fünften Peptidfragments, das die Aminosäuresequenz QAAKEFIAWLVKX³⁵R (SEQ ID NR.: 12) umfasst, wobei die Reste der Sequenz gegebenenfalls Seitenkettenschutz umfassen; und
f) Koppeln des fünften Fragments an das dritte Fragment, gefolgt von der Entfernung der Seitenkettenschutzgruppen, zur Bereitstellung eines insulinotropen Peptids der Formel HX⁸EX¹⁰TFTSDVSSYLEGQAAKEFIAWLVKX³⁵R (SEQ ID NR.: 5) und Gegenstücken davon, wobei X⁸ und X³⁵ Aminosäurereste sind, die Aib entsprechen, und X¹⁰ ein Aminosäurerest ist, der Glycin entspricht.

4. Verfahren nach den Ansprüchen 1 oder 3, wobei X⁸ ein Aminosäurerest ist, der Methylalanin entspricht.

5. Verfahren nach den Ansprüchen 1 oder 3, wobei X¹⁰ ein Aminosäurerest ist, der Glycin entspricht.

6. Peptidfragment mit der Aminosäuresequenz HX⁸EX¹⁰ (SEQ ID NR.: 6), wobei X⁸ und X¹⁰ jeweils Reste einer achiralen Aminosäure sind, wobei jeder von H, E, X⁸ und X¹⁰ gegebenenfalls Seitenkettenschützung umfasst.

7. Peptidfragment nach Anspruch 6, wobei X⁸ ein Aminosäurerest ist, der Aib entspricht, und X¹⁰ ein Aminosäurerest ist, der Glycin entspricht.

8. Verfahren nach Anspruch 1, wobei das insulinotrope Peptid die Aminosäuresequenz (SEQ ID NR.: 5)
HX⁸EX¹⁰TFTSDVSSYLEGQAAKEFIAWLVKX³⁵R
und Gegenstücke davon umfasst, wobei jedes der Symbole X an den Positionen 8, 10 und 35 unabhängig einen achiralen, gegebenenfalls sterisch gehinderten Aminosäurerest bezeichnet; und wobei einer oder mehrere der Aminosäurereste gegebenenfalls Seitenkettenschützung umfassen.

9. Verfahren nach Anspruch 8, wobei mindestens einer von X⁸ und X³⁵ ein Rest von Aib ist.

10. Verfahren nach Anspruch 8, wobei X¹⁰ ein Rest von Glycin ist.

11. Verfahren nach Anspruch 1, wobei X¹⁷⁻¹⁸ die Formel 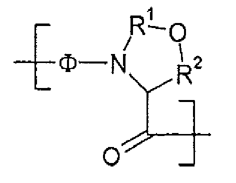 aufweist, wobei φ den Rest irgendeiner Aminosäure darstellt, der gegebenenfalls Seitenkettenschützung umfasst, und jeder von R¹ und R² unabhängig eine geeignete zweiwertige Verknüpfungskomponente ist.

12. Verfahren nach Anspruch 11, wobei φ einen Rest von Ser darstellt, der gegebenenfalls Seitenkettenschützung umfasst.

13. Verfahren nach Anspruch 11, wobei R² -CH₂- ist.

14. Verfahren nach Anspruch 11, wobei R¹ 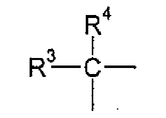 ist, wobei jeder von R³ und R⁴ unabhängig eine einwertige Komponente, ausgewählt aus H oder Niederalkyl, ist; oder R³ und R⁴ auch weitere Glieder einer Ringstruktur sein können.

15. Verfahren nach Anspruch 14, wobei jeder von R³ und R⁴ Methyl ist.

16. Peptid oder ein Gegenstück davon, das die Aminosäuresequenz TFTSDVX¹⁷⁻¹⁸YLEG (SEQ ID NR.: 8) umfasst, wobei der Rest, der mit dem Symbol X¹⁷⁻¹⁸ bezeichnet wird, ein Dipeptidrest eines Pseudoprolins ist; wobei die Aminosäurereste gegebenenfalls Seitenkettenschützung umfassen.

17. Verfahren nach Anspruch 1, wobei X³⁵ ein Aminosäurerest von Methylalanin ist.

18. Verfahren nach den Ansprüchen 1 bis 2, wobei das insulinotrope Peptid die Aminosäuresequenz (SEQ ID NR.: 4)
HAibEGTFTSDVSSYLEGQAAKEFIAWLVKAibR
und Gegenstücke davon aufweist.

19. Verfahren nach Anspruch 18, wobei das insulinotrope Peptid die Aminosäuresequenz (SEQ ID NR.: 4)
HAibEGTFTSDVSSYLEGQAAKEFIAWLVKAibR
und Gegenstücke davon aufweist, die an dem C-Terminus amidiert ist.

## Revendications

1. Procédé pour la préparation d'un peptide insulinotrope, comprenant les étapes a) à f) suivantes :
a) fournir un premier fragment peptidique comprenant la séquence d'aminoacides HX⁸EX¹⁰ (SEQ. ID N° 6), dans laquelle X⁸ et X¹⁰ représentent chacun le résidu d'un aminoacide achiral, chacun de H et E comprenant facultativement une protection de chaîne latérale ;
b) fournir un deuxième fragment peptidique comprenant la séquence d'aminoacides TFTSDVX¹⁷⁻¹⁸YLEG (SEQ. ID N° 8) dans laquelle le résidu désigné par le symbole X¹⁷⁻¹⁸ est un résidu dipeptide d'une pseudoproline, lesdits résidus d'aminoacides de la séquence comprenant facultativement une protection de chaîne latérale ;
c) coupler le premier fragment au deuxième fragment pour fournir un troisième fragment peptidique comprenant la séquence d'aminoacides HX⁸EX¹⁰ TFTSDVX¹⁷⁻¹⁸YLEG (SEQ. ID N° 11), lesdits résidus d'aminoacides de la séquence comprenant facultativement une protection de chaîne latérale ;
d) fournir un quatrième fragment peptidique comprenant la séquence d'aminoacides QAAKEFIAWLVKX³⁵ (SEQ. ID N° 9), dans laquelle X³⁵ représente un résidu d'un aminoacide achiral, lesdits résidus d'aminoacides de la séquence comprenant facultativement une protection de chaîne latérale :
e) coupler le quatrième fragment peptidique à l'arginine afin de fournir un cinquième fragment peptidique comprenant la séquence d'aminoacides QAAKEFIAWLVKX³⁵R (SEQ. ID N° 12), lesdits résidus de la séquence comprenant facultativement une protection de chaîne latérale ; et
f) coupler le cinquième fragment au troisième fragment afin de fournir un peptide insulinotrope comprenant la séquence d'aminoacides HX⁸EX¹⁰TFTSDVX¹⁷⁻¹⁸YLEGQAAKEFIAWLVKX³⁵R (SEQ. ID N° 13), lesdits résidus de la séquence comprenant facultativement une protection de chaîne latérale.

2. Procédé suivant la revendication 1, comprenant une étape supplémentaire consistant :
g) à éliminer les groupes protecteurs de chaîne latérale afin de fournir un peptide insulinotrope comprenant la séquence d'aminoacides HX⁸EX¹⁰TFTSDVSSYLEGQAAKEFIAWLVKX³⁵R (SEQ. ID N° 5) et ses analogues, où chacun des symboles X aux positions 8, 10 et 35 représente indépendamment un résidu d'aminoacide achiral, facultativement à encombrement stérique.

3. Procédé de fabrication d'un peptide insulinotrope suivant la revendication 1, comprenant les étapes consistant :
a) à fournir un premier fragment peptidique comprenant la séquence d'aminoacides HX⁸EX¹⁰ (SEQ. ID N° 6), dans laquelle X⁸ représente un résidu d'aminoacide correspondant à Aib et X¹⁰ représente un résidu d'aminoacide correspondant à la glycine, et chacun de H et E comprend facultativement une protection de chaîne latérale ;
b) à fournir un deuxième fragment peptidique comprenant la séquence d'aminoacides TFTSDVX¹⁷⁻¹⁸YLEG (SEQ. ID N° 8), dans laquelle le résidu désigné par le symbole X¹⁷⁻¹⁸ est un résidu dipeptide d'une pseudoproline, lesdits résidus d'aminoacides de la séquence comprenant facultativement une protection de chaîne latérale ;
c) à coupler le premier fragment au deuxième fragment pour fournir un troisième fragment peptidique comprenant la séquence d'aminoacides HX⁸EX¹⁰TFTSDV X¹⁷⁻¹⁸YLEG (SEQ. ID N° 11), lesdits résidus d'aminoacides de la séquence comprenant facultativement une protection de chaîne latérale ;
d) à fournir un quatrième fragment peptidique comprenant la séquence d'aminoacides QAAKEFIAWLVKX³⁵ (SEQ. ID N° 9), dans laquelle X³⁵ est un résidu d'aminoacide correspondant à Aib, lesdits résidus d'aminoacides de la séquence comprenant facultativement une protection de chaîne latérale ;
e) à coupler le quatrième fragment peptidique à l'arginine afin de fournir un cinquième fragment peptidique comprenant la séquence d'aminoacides QAAKEFIAWLVKX³⁵R (SEQ. ID N° 12), lesdits résidus de la séquence comprenant facultativement une protection de chaîne latérale ; et
f) à coupler le cinquième fragment au troisième fragment, puis à éliminer les groupes protecteurs de chaîne latérale afin de fournir un peptide insulinotrope de formule HX⁸EX¹⁰TFTSDVSSYLEGQAAKEFIAWLVKX³⁵R (SEQ. ID N° 5) et ses analogues, où X⁸ et X³⁵ représentent des résidus d'aminoacides correspondant à Aib et X¹⁰ représente un résidu d'aminoacide correspondant à la glycine.

4. Procédé suivant la revendication 1 ou 3, dans lequel X⁸ représente un résidu d'aminoacide correspondant à la méthylalanine.

5. Procédé suivant la revendication 1 ou 3, dans lequel X¹⁰ représente un résidu d'aminoacide correspondant à la glycine.

6. Fragment peptidique ayant la séquence d'aminoacides HX⁸EX¹⁰ (SEQ. ID N° 6), dans laquelle X⁸ et X¹⁰ représentent chacun le résidu d'un aminoacide achiral, chacun de H, E, X⁸ et X¹⁰ comprenant facultativement une protection de chaîne latérale.

7. Fragment peptidique suivant la revendication 6, dans lequel X⁸ représente un résidu d'aminoacide correspondant à Aib et X¹⁰ représente un résidu d'aminoacide correspondant à la glycine.

8. Procédé suivant la revendication 1, dans lequel le peptide insulinotrope comprend la séquence d'aminoacides (SEQ. ID N° 5)
HX⁸EX¹⁰TFTSDVSSYLEGQAAKEFIAWLVKX³⁵R
et ses analogues, où chacun des symboles X aux positions 8, 10 et 35 représente indépendamment un résidu d'aminoacide achiral, facultativement à encombrement stérique ; et où un ou plusieurs des résidus d'aminoacides comprennent facultativement une protection de chaîne latérale.

9. Procédé suivant la revendication 8, dans lequel au moins un de X⁸ et X³⁵ représente un résidu Aib.

10. Procédé suivant la revendication 8, dans lequel X¹⁰ représente un résidu glycine.

11. Procédé suivant la revendication 1, dans lequel X¹⁷⁻¹⁸ répond à la formule 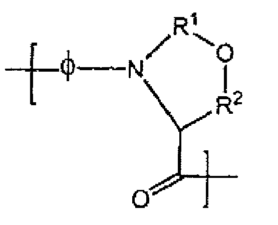 dans laquelle Φ représente le résidu de n'importe quel aminoacide comprenant facultativement une protection de chaîne latérale et chacun de R¹ et R² représente indépendamment un groupement de liaison divalent convenable.

12. Procédé suivant la revendication 11, dans lequel Φ représente un résidu Ser comprenant facultativement une protection de chaîne latérale.

13. Procédé suivant la revendication 11, dans lequel R² représente un groupe -CH₂-.

14. Procédé suivant la revendication 11, dans lequel R¹ représente un groupe 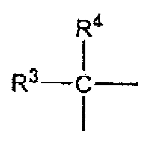 dans lequel chacun de R³ et R⁴ représente indépendamment un groupement monovalent choisi entre H et un groupement alkyle inférieur ; ou bien R³ et R⁴ peuvent également être des co-membres d'une structure cyclique.

15. Procédé suivant la revendication 14, dans lequel chacun de R³ et R⁴ représente un groupe méthyle.

16. Peptide ou un de ses analogues comprenant la séquence d'aminoacides TFTSDVX¹⁷⁻¹⁸YLEG (SEQ. ID N° 8) dans laquelle le résidu désigné par le symbole X¹⁷⁻¹⁸ est un résidu dipeptidique d'une pseudoproline ; lesdits résidus d'aminoacides comprenant facultativement une protection de chaîne latérale.

17. Procédé suivant la revendication 1, dans lequel X³⁵ représente un résidu d'aminoacide méthylalanine.

18. Procédé suivant la revendication 1 ou 2, dans lequel le peptide insulinotrope a la séquence d'aminoacides (SEQ. ID N° 4)
HAibEGTFTSDVSSYLEGQAAKEFIAWLVKAibR
et ses analogues.

19. Procédé suivant la revendication 18, dans lequel le peptide insulinotrope a la séquence d'aminoacides (SEQ. ID N° 4)
HAibEGTFTSDVSSYLEGQAAKEFIAWLVKAibR
et ses analogues, la séquence étant amidée à l'extrémité C-terminale.


### SEQUENCE LISTING

<110> F. Hoffmann-La Roche AG
   Rober t s, Christopher R
<120> INSULINOTROPIC PEPTIDE SYNTHESIS USING SOLID AND SOLUTION PHASE
   CONBINATION TECHNIQUES
<130> Case 23831
<150> US 60/ 815, 919
   <151> 2006-06-23
<160> 13
<170> Patent Inversion 3. 4
<210> 1
   <211> 37
   <212> PRT
   <213> Homo sapi ens
<400> 1
   His Asp Glu Phe Glu Arg His Ala Glu Gly Thr Phe Thr Ser Asp Val
   1               5                   10                  15
   Ser Ser Tyr Leu Glu Gly Gln Ala Ala Lys Glu Phe Ile Ala Trp Leu
               20                  25                  30
   Val Lys Gly Arg Gly
           35
<210> 2
   <211> 31
   <212> PRT
   <213> Homosapiens
<400> 2
   His Ala Glu Gly Thr Phe Thr Ser Asp Val Ser Ser Tyr Leu Glu Gly
   1               5                   10                  15
   Gln Ala Ala Lys Glu Phe Ile Ala Trp Leu Val Lys Gly Arg Gly
               20                  25                  30
<210> 3
   <211> 30
   <212> PRT
   <213> Homosapiens
<400> 3
   His Ala Glu Gly Thr Phe Thr Ser Asp Val Ser Ser Tyr Leu Glu Gly
   1               5                   10                  15
   Gln Ala Ala Lys Glu Phe Ile Ala Trp Leu Val Lys Gly Arg
               20                  25                  30
<210> 4
   <211> 30
   <212> PRT
   <213> Artificial sequence
<220>
   <223> Chemically synthesized peptide fragment
<220>
   <221> M SC_FEATURE
   <222> (2)..(2)
   <223> Xaa is Aib
<220>
   <221> M SC_FEATURE
   <222> (29)..(29)
   <223> Xaa is Aib
<400> 4
   His Xaa Glu Gly Thr Phe Thr Ser Asp Val Ser Ser Tyr Leu Glu Gly
   1               5                   10                  15
   Gln Ala Ala Lys Glu Phe Ile Ala Trp Leu Val Lys Xaa Arg
               20                  25                  30
<210> 5
   <211> 30
   <212> PRT
   <213> Artificial sequence
<220>
   <223> Chemically synthesized peptide fragment
<220>
   <221> M SC_FEATURE
   <222> (2)..(2)
   <223> Xaa is an achiral amino acid
<220>
   <221> M SC_FEATURE
   <222> (4)..(4)
   <223> Xaa is an achiral amino acid
<220>
   <221> M SC_FEATURE
   <222> (29)..(29) achiral amino acid
   <223> Xaa is an achiral amino acid
<400> 5
   His Xaa Glu Xaa Thr Phe Thr Ser Asp Val Ser Ser Tyr Leu Glu Gly
   1 5 10 15
   Gln Ala Ala Lys Glu Phe Ile Ala Trp Leu Val Lys Xaa Arg
   20 25 30
<210> 6
   <211> 4
   <212> PRT
   <213> Artificial sequence
<220>
   <223> Chemically synthesized peptide fragment
<220>
   <221> M SC_FEATURE
   <222> (2)..(2)
   <223> Xaa is an achiral amino acid
<220>
   <221> M SC_FEATURE
   <222> (4)..(4)
   <223> Xaa is an achiral amino acid
<400> 6
   His Xaa Glu Xaa 1
<210> 7
   <211> 4
   <212> PRT
   <213> Artificial sequence
<220>
   <223> Chemically synthesized peptide fragment
<220>
   <221> M SC_FEATURE
   <222> (2)..(2)
   <223> Xaa i s Aib
<400> 7
   Hi s Xaa Glu Gly 1
<210> 8
   <211> 12
   <212> PRT
   <213> Artificial sequence
<220>
   <223> Chemically synthesized peptide fragment
<220>
   <221> M SC_FEATURE
   <222> (7)..(7)
   <223> Xaa is an amino acid of a pseudoproline dipeptide (residues 7- 8)
<220>
   <221> M SC_FEATURE
   <222> (8)..(8)
   <223> Xaa is an amino acid of a pseudoproline dipeptide (residues 7- 8)
<400> 8
   Thr Phe Thr Ser Asp Val Xaa Xaa Tyr Leu Glu Gly
   1               5                   10
<210> 9
   <211> 13
   <212> PRT
   <213> Artificial sequence
<220>
   <223> Chemically synthesized peptide fragment
<220>
   <221> M SC_FEATURE
   <222> (13)..(13)
   <223> Xaa is an achiral amino acid
<400> 9
   Gln Ala Ala Lys Glu Phe Ile Ala Trp Leu Val Lys Xaa
   1               5                   10
<210> 10
   <211> 13
   <212> PRT
   <213> Artificial sequence
<220>
   <223> Chemically synthesized peptide fragment
<220>
   <221> M SC_FEATURE
   <222> (13)..(13)
   <223> Xaa is Aib
<400> 10
   Gln Ala Ala Lys Glu Phe Ile Ala Trp Leu Val Lys Xaa
   1 5 10
<210> 11
   <211> 16
   <212> PRT
   <213> Artificial sequence
<220>
   <223> Chemically synthesized peptide fragment
<220>
   <221> M SC_FEATURE
   <222> (2)..(2)
   <223> Xaa is an achiral amino acid
<220>
   <221> M SC_FEATURE
   <222> (4)..(4) achiral amino acid
   <223> Xaa is an achiral amino acid
<220>
   <221> M SC_FEATURE
   <222> (11)..(11)
   <223> Xaa is an amino acid of a pseudoproline dipeptide (residues 11-12)
<220>
   <221> M SC_FEATURE
   <222> (12)..(12)
   <223> Xaa is an amino acid of a pseudoproline dipeptide (residues 11-12)
<400> 11
   His Xaa Glu Xaa Thr Phe Thr Ser Asp Val Xaa Xaa Tyr Leu Glu Gly
   1 5 10 15
<210> 12
   <211> 14
   <212> PRT
   <213> Artificial sequence
<220>
   <223> Chemically synthesized peptide fragment
<220>
   <221> M SC_FEATURE
   <222> (13)..(13)
   <223> Xaa is an achiral amino acid
<400> 12
   Gln Ala Ala Lys Glu Phe Ile Ala Trp Leu Val Lys Xaa Arg
   1 5 10
<210> 13
   <211> 30
   <212> PRT
   <213> Artificial sequence
<220>
   <223> Chemically synthesized peptide fragment
<220>
   <221> M SC_FEATURE
   <222> (2).. (2)
   <223> Xaa is an achiral amino acid
<220>
   <221> M SC_FEATURE
   <222> (4)..(4)
   <223> Xaa is an achiral amino acid
<220>
   <221> M SC_FEATURE
   <222> (11)..(11)
   <223> Xaa is an amino acid of a pseudoproline dipeptide (residues 11-12)
<220>
   <221> M SC_FEATURE
   <222> (12)..(12)
   <223> Xaa is an amino acid of a pseudoproline dipeptide (residues 11-12)
<220>
   <221> M SC_FEATURE
   <222> (29)..(29)
   <223> Xaa is an achiral amino acid
<400> 13
   His Xaa Glu Xaa Thr Phe Thr Ser Asp Val Xaa Xaa Tyr Leu Glu Gly
   1 5 10 15
   Gln Ala Ala Lys Glu Phe Ile Ala Trp Leu Val Lys Xaa Arg
   20 25 30